# EUROPEAN PATENT SPECIFICATION

(11) **EP 2 254 851 B1**
(45) Date of publication and mention of the grant of the patent: **26.10.2016**
(21) Application number: 09711602.4
(22) Date of filing: 19.02.2009
(51) Int. Cl.: C07C 17/383, C07C 17/386, C07C 21/18, C01B 7/19, C07C 17/38

(54) **AZEOTROPE COMPOSITIONS COMPRISING 3,3,3-TRIFLUOROPROPENE AND HYDROGEN FLUORIDE AND PROCESSES FOR SEPARATION THEREOF**
3,3,3-TRIFLUORPROPEN UND WASSERSTOFFFLUORID ENTHALTENDE AZEOTROPE ZUSAMMENSETZUNGEN UND VERFAHREN ZU DEREN TRENNUNG
COMPOSITIONS AZEOTROPIQUE COMPOSEE DE 3,3,3-TRIFLUOROPROPENE ET DE FLUORURE D'HYDROGENE ET PROCEDES DE SEPARATION

(30) Priority: 21.02.2008 US 30365 P
(43) Date of publication of application: 01.12.2010
(73) Proprietor: The Chemours Company FC, LLC, Wilmington DE 19801 (US)
(72) Inventor: KNAPP, Jeffrey, P., Wilmington Delaware 19808 (US)
(74) Representative: Matthews, Derek Peter
(86) International application number: PCT/US2009/034478
(87) International publication number: WO 2009/105517

(56) References cited:
- WO-A-2008/024508
- US-A1- 2006 106 263
- US-A1- 2007 100 173
- US-A1- 2007 100 175

## Description

### BACKGROUND INFORMATION

### Field of the Disclosure

This disclosure relates in general to processes for separating HF from fluoroolefins.

### Description of the Related Art

The chemical manufacture of fluoroolefins may produce mixtures of the desired fluoroolefins and hydrogen fluoride (HF). The separation of fluoroolefins and HF is not always easily accomplished. Existing methods of distillation and decantation are very often ineffective for separation of these compounds. Aqueous scrubbing may be effective, but requires the use of large amounts of scrubbing solutions and produces excessive waste as well as wet product that must then be dried. Therefore, there is a need for new methods of separating HF from fluoroolefins.

US 2006/0106263 A1 discloses processes for producing hydrofluoroolefins, and processes for separating hydrofluoroolefins from hydrofluorocarbons and hydrogen fluoride by azeotropic distillation. One hydrofluoroolefin referred to therein is HFC-1243zf (3,3,3-trifluoro-propene), but no specific azeotropes are disclosed.

### SUMMARY

In one embodiment, the present disclosure provides a process for separating a mixture comprising HF and HFC-1243zf, said process comprising: a) feeding the composition comprising HF and HFC-1243zf to a first distillation column; b) removing an azeotropic or azeotrope-like composition consisting essentially of from 67.2 to 82.5 mole percent HFC-1243zf and from 32.8 to 17.5 mole percent HF, said composition having a vapor pressure from 8.0 psia (55.2 kPa) to 817 psia (5633 kPa) at a temperature from -40°C to 110°C, as a first distillate and either i) HF or ii) HFC-1243zf as a first column bottoms composition; c) condensing the first distillate to form 2 liquid phases, being i) an HF-rich phase and ii) an HFC-1243zf-rich phase; and d) recycling a first liquid phase enriched in the same compound that is removed as the first column bottoms, said first liquid phase being either i) HF-rich phase or ii) HFC-1243zf-rich phase, back to the first distillation column.

In another embodiment, the present disclosure provides a process for separating HFC-1243zf from a mixture comprising hydrogen fluoride and said HFC-1243zf, wherein said HFC-1243zf is present in a concentration greater than the azeotrope concentration for hydrogen fluoride and said HFC-1243zf, said process comprising: a) feeding said mixture comprising hydrogen fluoride and said HFC-1243zf to a first distillation column; b) removing an azeotrope or azeotrope-like composition as defined above as a first distillate from the first distillation column; c) recovering HFC-1243zf essentially free of hydrogen fluoride from the bottom of the first distillation column; d) condensing the azeotrope composition to form two liquid phases, being i) a hydrogen fluoride-rich phase and ii) an HFC-1243zf-rich phase; and e) recycling the HFC-1243zf-rich phase to the first distillation column.

In another embodiment, the present disclosure provides a process for separating hydrogen fluoride from a mixture comprising hydrogen fluoride and HFC-1243zf, wherein hydrogen fluoride is present in a concentration greater than the azeotrope concentration for hydrogen fluoride and said HFC-1243zf, said process comprising: a) feeding said mixture comprising hydrogen fluoride and HFC-1243zf to a first distillation column; b) removing an azeotrope or azeotrope-like composition as defined above as a distillate from the first distillation column; c) recovering hydrogen fluoride essentially free of HFC-1243zf from the bottom of the first distillation column; d) condensing the azeotrope composition to form two liquid phases, being an HFC-1243zf-rich phase and a hydrogen fluoride-rich phase; and e) recycling the HF-rich phase to the first distillation column.

In another embodiment, the present disclosure provides a process for the separation of HFC-1243zf from a mixture of HFC-1243zf, HF, and at least one of HFC-254fb or HFC-254eb, said process comprising: a) subjecting said mixture to a first distillation step, wherein additional HFC-1243zf is fed from a second distillation step, to form a first distillate comprising an azeotrope of HFC-1243zf and HF and a first bottoms composition comprising at least one of HFC-254fb or HFC-254eb; b) feeding said first distillate to a second distillation step to form a second distillate comprising an azeotrope of HFC-1243zf and HF and a second bottoms composition comprising HFC-1243zf essentially free of HF; c) condensing said second distillate to form two liquid phases, being i) an HF-rich phase and ii) an HFC-1243zf-rich phase; and d) recycling the HFC-1243zf-rich phase from (c) back to the first distillation step.

The foregoing general description and the following detailed description are exemplary and explanatory only and are not restrictive of the invention, as defined in the appended claims.

The entrainer-based processes described below have been found to lack unity with the azeotrope-based processes and are not claimed herein and are not part of the invention.

### BRIEF DESCRIPTION OF THE DRAWINGS

Embodiments are illustrated in the accompanying figures to improve understanding of concepts as presented herein.
FIG. 1 is an illustration of one embodiment of an azeotropic distillation for the separation of HF and HFC-1243zf with no added entrainer.
FIG. 2 is an illustration of one embodiment of an azeotropic distillation for the separation of HF and HFC-1243zf with an added entrainer.
FIG. 3 is an illustration of one embodiment of a process to separate at least one of HFC-254eb and HFC-254fb from a mixture comprising HFC-1243zf, HF and said at least one of HFC-254eb and HFC-254fb via azeotropic distillation wherein HFC-1243zf acts as an entrainer followed by a process in which HFC-1243zf and HF are separated from a mixture comprising HFC-1243zf and HF, but now substantially free of HFC-254eb and/or HFC-254fb, by azeotropic distillation without the addition of another chemical compound to function as an entrainer.
FIG. 4 is an illustration of one embodiment of a process to separate HFC-1243zf and at least one of HFC-254eb and HFC-254fb from a mixture comprising HFC-1243zf, HF and said at least one of HFC-254eb and HFC-254fb via azeotropic distillation wherein a supplemental entrainer is fed to the distillation.
FIG 5 is an illustration of one embodiment of a process to separate at least one of HFC-254eb and HFC-254fb from a mixture comprising HFC-1243zf, HF and said at least one of HFC-254eb and HFC-254fb via azeotropic distillation wherein HFC-1243zf acts as an entrainer followed by a process in which HFC-1243zf and HF are separated from a mixture comprising HFC-1243zf and HF, but now substantially free of HFC-254eb and/or HFC-254fb, by azeotropic distillation with an added entrainer.
FIG 6 illustrates another embodiment of the process shown in Figure 3 wherein the two-phase mixture leaving the condenser of the first column is decanted and separated into HFC-1243zf-rich and HF-rich streams which are fed to the HFC-1243zf and HF columns, respectively.
FIG 7 illustrates another embodiment of the process shown in Figure 5 wherein the two-phase mixture leaving the condenser of the first column is decanted and separated into HFC-1243zf-rich and HF-rich streams which are fed to the HFC-1243zf and HF columns, respectively.
FIG 8 illustrates another embodiment of the process shown in FIG 6, wherein the three columns, 20, 110, and 220, share one decanter.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

### 1. Definitions and Clarification of Terms

Before addressing details of embodiments described below, some terms are defined or clarified.

Binary azeotropic or azeotrope-like compositions of substantially constant-boiling mixtures can be characterized, depending upon the conditions chosen, in a number of ways. For example, it is well known by those skilled in the art, that, at different pressures the composition of a given azeotrope or azeotrope-like composition will vary at least to some degree, as will the boiling point temperature. Thus, an azeotropic or azeotrope-like composition of two compounds represents a unique type of relationship but with a variable composition that depends on temperature and/or pressure. Therefore, compositional ranges, rather than fixed compositions, are often used to define azeotropes and azeotrope-like compositions.

By "azeotropic" composition is meant a constant boiling liquid admixture of two or more substances that behaves as a single substance. One way to characterize an azeotropic composition is that the vapor produced by partial evaporation or distillation of the liquid has the same composition as the liquid from which it was evaporated or distilled, that is, the admixture distills/refluxes without compositional change. Constant boiling compositions are characterized as azeotropic because they exhibit either a maximum or minimum boiling point, as compared with that of the non-azeotropic mixtures of the same components. Azeotropic compositions are also characterized by a minimum or a maximum in the vapor pressure of the mixture relative to the vapor pressure of the neat components at a constant temperature.

By "azeotrope-like" composition (sometimes referred to as "near-azeotropic") is meant a constant boiling, or substantially constant boiling, liquid admixture of two or more substances that behaves as a single substance. One way to characterize an azeotrope-like composition is that the vapor produced by partial evaporation or distillation of the liquid has substantially the same composition as the liquid from which it was evaporated or distilled, that is, the admixture distills/refluxes without substantial composition change. Another way to characterize an azeotrope-like composition is that the bubble point vapor pressure and the dew point vapor pressure of the composition at a particular temperature are substantially the same. An azeotrope-like composition can also be characterized by the area that is adjacent to the maximum or minimum vapor pressure in a plot of composition vapor pressure at a given temperature as a function of mole fraction of components in the composition.

It is recognized in the art that a composition is azeotrope-like if, after 50 weight percent of the composition is removed such as by evaporation or boiling off, the difference in vapor pressure between the original composition and the composition remaining after 50 weight percent of the original composition has been removed is less than about 10 percent, when measured in absolute units. By absolute units, it is meant measurements of pressure and, for example, psia, atmospheres, bars, torr, dynes per square centimeter, millimeters of mercury, inches of water and other equivalent terms well known in the art. If an azeotrope is present, there is no difference in vapor pressure between the original composition and the composition remaining after 50 weight percent of the original composition has been removed.

For compositions that are azeotropic, there is usually some range of compositions around the azeotrope point that, for a maximum boiling azeotrope, have boiling points at a particular pressure higher than the pure components of the composition at that pressure and have vapor pressures at a particular temperature lower than the pure components of the composition at that temperature, and that, for a minimum boiling azeotrope, have boiling points at a particular pressure lower than the pure components of the composition at that pressure and have vapor pressures at a particular temperature higher than the pure components of the composition at that temperature. Boiling temperatures and vapor pressures above or below that of the pure components are caused by unexpected intermolecular forces between and among the molecules of the compositions, which can be a combination of repulsive and attractive forces such as van der Waals forces and hydrogen bonding. The range of compositions that have a maximum or minimum boiling point at a particular pressure, or a maximum or minimum vapor pressure at a particular temperature, may or may not be coextensive with the range of compositions that have a change in vapor pressure of less than about 10% when 50 weight percent of the composition is evaporated. In those cases where the range of compositions that have maximum or minimum boiling temperatures at a particular pressure, or maximum or minimum vapor pressures at a particular temperature, are broader than the range of compositions that have a change in vapor pressure of less than about 10% when 50 weight percent of the composition is evaporated, the unexpected intermolecular forces are nonetheless believed important in that the refrigerant compositions having those forces that are not substantially constant boiling may exhibit unexpected increases in the capacity or efficiency versus the components of the refrigerant composition.

It is recognized in the art that both the boiling point and the amount of each component of an azeotropic composition can change when the azeotrope liquid composition is subjected to boiling at different pressures. Thus, an azeotropic composition may be defined in terms of the unique relationship that exists among components or in terms of the exact amounts of each component of the composition characterized by a fixed boiling point at a specific pressure. An azeotrope or azeotrope-like composition of two compounds can be characterized by defining compositions characterized by a boiling point at a given pressure, thus providing identifying characteristics without unduly limiting the scope of the invention by a specific numerical composition, which is limited by and is only as accurate as the analytical equipment available.

It is recognized in this field that when the relative volatility of a system approaches 1.0, the system is defined as forming an azeotrope-like composition. Relative volatility is the ratio of the volatility of component 1 to the volatility of component 2. The ratio of the mole fraction of a component in vapor to that in liquid is the volatility of the component.

To determine the relative volatility of any two compounds, a method known as the PTx method can be used. In this procedure, the total absolute pressure in a cell of known volume is measured at a constant temperature for various compositions of the two compounds. Use of the PTx Method is described in detail in "Phase Equilibrium in Process Design", Wiley-Interscience Publisher, 1970, written by Harold R. Null, on pages 124 to 126.

These measurements can be converted into equilibrium vapor and liquid compositions in the PTx cell by using an activity coefficient equation model, such as the Non-Random, Two-Liquid (NRTL) equation, to represent liquid phase nonidealities. Use of an activity coefficient equation, such as the NRTL equation is described in detail in "The Properties of Gases and Liquids," 4th edition, published by McGraw Hill, written by Reid, Prausnitz and Poling, on pages 241 to 387, and in "Phase Equilibria in Chemical Engineering," published by Butterworth Publishers, 1985, written by Stanley M. Walas, pages 165 to 244. Without wishing to be bound by any theory or explanation, it is believed that the NRTL equation, together with the PTx cell data, can sufficiently predict the relative volatilities of the HFC-1243zf-containing compositions of the present invention and can therefore predict the behavior of these mixtures in multistage separation equipment such as distillation columns.

As used herein, the term "azeotrope" is meant to refer to azeotrope compositions and/or azeotrope-like compositions.

The process equipment for all the processes disclosed herein and the associated feed lines, effluent lines and associated units may be constructed of materials resistant to hydrogen fluoride. Typical materials of construction, well-known to the art, include stainless steels, in particular of the austenitic type, and the well-known high nickel alloys such as Monel® nickel-copper alloys, Hastelloy® nickel based alloys and Inconel® nickel-chromium alloys.

By azeotropic distillation is meant a process in which a distillation column is operated under conditions to cause one or more azeotropic or azeotrope-like composition to form, and thereby facilitates the separation of the components of the mixture. Azeotropic distillations may occur where only the components of the mixture to be separated are distilled, or where an entrainer is added that forms an azeotrope with one or more of the components of the initial mixture. Entrainers that act in this manner, that is to say, that form an azeotrope with one of more of the components of the mixture to be separated thus facilitating the separation of those components by distillation, are more commonly called azeotroping agents or azeotropic entrainers.

In conventional or azeotropic distillations, the overhead or distillate stream exiting the column may be condensed using conventional reflux condensers. At least a portion of this condensed stream can be returned to the top of the column as reflux, and the remainder recovered as product or for optional processing. The ratio of the condensed material which is returned to the top of the column as reflux to the material removed as distillate is commonly referred to as the reflux ratio. The compounds and entrainer exiting the column as distillate or distillation bottoms stream can then be passed to a stripper or second distillation column for separation by using conventional distillation, or may be separated by other methods, such as decantation. If desired, the entrainer may then be recycled back to the first distillation column for reuse.

The specific conditions which can be used for practicing the invention depend upon a number of parameters, such as the diameter of the distillation column, feed points, number of separation stages in the column, among others. In one embodiment, the operating pressure of the distillation system may range from about 5 to 500 psia 0.5 to 50.5 MPa; in another embodiment, about 20 to 400 psia 2 to 40.5 MPa. Normally, increasing the reflux ratio results in increased distillate stream purity, but generally the reflux ratio ranges between 1/1 to 200/1. The temperature of the condenser, which is located adjacent to the top of the column, is normally sufficient to substantially fully condense the distillate that is exiting from the top of the column, or is that temperature required to achieve the desired reflux ratio by partial condensation.

The problems associated with conventional distillation can be solved by a distillation process using entrainers. The difficulty in applying this method is that there is no known way, short of experimentation, of predicting which if any compound will be an effective entrainer.

As used herein, by "essentially free of" is meant that a composition contains less than about 100 ppm (mole basis), less than about 10 ppm or less than about 1 ppm, of the specified component. If a composition is essentially free of more than one component, then the total concentration of those components is less than about 100 ppm, less than about 10 ppm, or less than about 1 ppm.

Hydrogen fluoride (HF, anhydrous) is a commercially available chemical or can be produced by methods known in the art.

By entrainer is meant any compound that, when added to a first mixture, forms one or more azeotropes with the components of the mixture to facilitate separation of the components of the mixture. As used herein, the terms "entrainer" and "entraining agent" are used interchangeably and are to be interpreted as having identical meaning.

The term "entrainer" is used herein to describe any compound that would be effective in separation of fluoroolefins from mixtures comprising HF and fluoroolefin in an azeotropic distillation process. Included as useful entrainers are those compounds that form azeotropes with one or more of the components of a mixture, including fluoroolefins, HF, and possible hydrofluorocarbons for which the boiling point of at least one of such azeotropes is lower than the boiling point of the fluoroolefin/HF azeotrope.

Entrainers may be selected from the group consisting of hydrocarbons, chlorocarbons, chlorofluorocarbons, hydrochlorofluorocarbons, hydrofluorocarbons, perfluorocarbons, fluoroethers, HFPO, SF₆, chlorine, hexafluoroacetone, and mixtures thereof.

Hydrocarbon entrainers comprise compounds containing 1 to 5 carbon atoms and hydrogen. Hydrocarbon entrainers may be linear, branched, cyclic, saturated or unsaturated compounds. Representative hydrocarbon entrainers include but are not limited to methane, ethane, ethylene, acetylene, vinylacetylene, propane, propylene, propyne, cyclopropane, cyclopropene, propadiene, n-butane, isobutane, 1-butene, isobutene, 1,3-butadiene, 2,2-dimethylpropane, cis-2-butene, trans-2-butene, 1-butyne, n-pentane, isopentane, neopentane, cyclopentane, 1-pentene, 2-pentene, and mixtures thereof.

Chlorocarbon entrainers comprise compounds containing carbon, chlorine and optionally hydrogen, including but not limited to methylene chloride (CH₂Cl₂), and methyl chloride (CH₃Cl).

Chlorofluorocarbon (CFC) entrainers comprise compounds with carbon, chlorine and fluorine. Representative CFCs include but are not limited to dichlorodifluoromethane (CFC-12), 2-chloro-1,1,2-trifluoroethylene, chloropentafluoroethane (CFC-115), 1,2-dichloro-1,1,2,2-tetrafluoroethane (CFC-114), 1,1-dichloro-1,2,2,2-tetrafluoroethane (CFC-114a), 1,1,2-trichloro-1,2,2-trifluoroethane (CFC-113), 1,1,1-trichloro-2,2,2-trifluoroethane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropene (CFC-1215xc), and mixtures thereof.

Hydrochlorofluorocarbon (HCFC) entrainers comprise compounds with carbon, chlorine, fluorine and hydrogen. Representative HCFCs include but are not limited to dichlorofluoromethane (HCFC-21), 1,1-dichloro-3,3,3-trifluoroethane (HCFC-123), 1,1-dichloro-1-fluoroethane (HCFC-141b), 2-chloro-1,1,1,2-tetrafluoroethane (HCFC-124), 1-chloro-1,1,2,2-tetrafluoroethane (HCFC-124a), 2-chloro-1,1,1-trifluoroethane (HCFC-133a), 1-chloro-1,1-difluoroethane (HCFC-142b), 2-chloro-1,1-difluoroethylene (HCFC-1122), and mixtures thereof.

Hydrofluorocarbon (HFC) entrainers comprise compounds that contain carbon, hydrogen and fluorine. Representative HFCs include but are not limited to 1,1,2-trifluoroethylene (HFC-1123), 1,1-difluoroethylene (HFC-1132a), 2,3,3,3-tetrafluoropropene (HFC-1234yf), and mixtures thereof.

Perfluorocarbon (PFC) entrainers comprise compounds with carbon and fluorine only. Representative PFCs include but are not limited to hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), octafluorocyclobutane (PFC-C318), decafluorobutane (PFC-31-10, any isomer(s)), 2,3-dichloro-1,1,1,4,4,4-hexafluoro-2-butene (PFC-1316mxx), octafluoro-2-butene (PFC-1318my, cis and trans), hexafluorobutadiene (PFC-2316), and mixtures thereof.

Fluoroether entrainers comprise compounds with carbon, fluorine, optionally hydrogen and at least one ether group oxygen. Representative fluoroethers include but are not limited to trifluoromethyl-difluoromethyl ether (CF₃OCHF₂, HFOC-125E), 1,1-difluorodimethyl ether, tetrafluorodimethylether (HFOC-134E), difluoromethyl methyl ether (CHF₂OCH₃, HFOC-152aE), pentafluoroethyl methyl ether, and mixtures thereof.

Miscellaneous other compounds that may be useful as entrainers include HFPO, chlorine (Cl₂), hexafluoroacetone, PMVE (perfluoromethylvinylether), PEVE (perfluoroethylvinylether), and mixtures thereof.

Entrainers as described above are available commercially or may be produced by methods known in the art.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below.

### 2. Azeotropic and Azeotrope-like compositions

Hydrogen fluoride (HF, anhydrous) is a commercially available chemical or can be produced by methods known in the art.

3,3,3-trifluoropropene (HFC-1243zf, CF₃CH=CH₂) may be prepared by known methods, such as dehydrofluorination of 1,1,1,2-tetrafluoropropane (CF₃CH₂CH₂F or HFC-254fb) or 1,1,1,3-tetrafluoropropane (CF₃CHFCH₃ or HFC-254eb). HFC-254fb is available commercially or may be made by the reaction of CIF with CCl₃CH₂CH₂Cl (N. N. Chuvatkin, et al, Zh. Org. Khim., 18 (1982) 946), by the fluorination of CF₃CH₂CH₂I with HgF, and via Zn reduction of CF₃CH₂CHFI (R. N. Hazeldine, et al, J. Chem. Soc., (1953) 1199) or CF₃CH₂CHFBr (P. Tarrant, et al, J. Am. Chem. Soc., 77 (1955) 2783). HFC-254eb is available commercially or may be made by the addition of fluoromethane and trifluoroethylene with an antimony pentafluoride catalyst, as described in for instance, US 6,184,426.

HFC-1243zf may also be made by fluorination of 1,1,1,3-tetrachloropropane (CCl₃CH₂CH₂Cl or HCC-250fb) with hydrogen fluoride over a fluorination catalyst such as chromium/alumina fluoride or chromium oxide catalysts. HCC-250fb may be made by processes known in the art such as described in US 4,605,802 and US 5,705,779 by an addition reaction of carbon tetrachloride and ethylene.

HFC-1243zf has been found to form a binary azeotropic composition with HF. The azeotropic composition comprises about 72.0 mole % HFC-1243zf and about 28.0 mole % HF at 29.8°C and 106.6 psia (735 kPa). Further, the azeotropic composition comprises about 76.2 mole % HFC1243zf and about 23.8 mole % HF at 79.7°C and 363 psia (2503 kPa).

For purposes of this invention, "effective amount" is defined as the amount of each component of the inventive compositions which, when combined, results in the formation of an azeotropic or azeotrope-like composition. This definition includes the amounts of each component, which amounts may vary depending on the pressure applied to the composition so long as the azeotropic or azeotrope-like compositions continue to exist at the different pressures, but with possible different boiling points. Therefore, effective amount includes the amounts, such as may be expressed in weight percentages, of each component of the compositions of the instant invention which form azeotropic or azeotrope-like compositions at temperatures or pressures other than as described herein.

For the purposes of this discussion, azeotropic or constant-boiling is intended to mean also essentially azeotropic or essentially-constant boiling. In other words, included within the meaning of these terms are not only the true azeotropes described above, but also other compositions containing the same components in different proportions, which are true azeotropes at other temperatures and pressures, as well as those equivalent compositions which are part of the same azeotropic system and are azeotrope-like in their properties. As is well recognized in this art, there is a range of compositions which contain the same components as the azeotrope, which will not only exhibit essentially equivalent properties for refrigeration and other applications, but which will also exhibit essentially equivalent properties to the true azeotropic composition in terms of constant boiling characteristics or tendency not to segregate or fractionate on boiling.

It is possible to characterize, in effect, a constant boiling admixture which may appear under many guises, depending upon the conditions chosen, by any of several criteria: The composition can be defined as an azeotrope of A, B, C (and D ... ) since the very term "azeotrope" is at once both definitive and limitative, and requires that effective amounts of A, B, C (and D ... ) for this unique composition of matter which is a constant boiling composition. It is well known by those skilled in the art, that, at different pressures, the composition of a given azeotrope will vary at least to some degree, and changes in pressure will also change, at least to some degree, the boiling point temperature. Thus, an azeotrope of A, B, C (and D ... ) represents a unique type of relationship but with a variable composition which depends on temperature and/or pressure. Therefore, compositional ranges, rather than fixed compositions, are often used to define azeotropes. The composition can be defined as a particular weight percent relationship or mole percent relationship of A, B, C (and D ... ), while recognizing that such specific values point out only one particular relationship and that in actuality, a series of such relationships, represented by A, B, C (and D ... ) actually exist for a given azeotrope, varied by the influence of pressure. An azeotrope of A, B, C (and D ... ) can be characterized by defining the compositions as an azeotrope characterized by a boiling point at a given pressure, thus giving identifying characteristics without unduly limiting the scope of the invention by a specific numerical composition, which is limited by and is only as accurate as the analytical equipment available.

The azeotrope or azeotrope-like compositions of the present invention can be prepared by any convenient method including mixing or combining the desired amounts. A preferred method is to weigh the desired component amounts and thereafter combine them in an appropriate container.

### 3. Separation process - Azeotropic distillation with no entrainer

It has been discovered that some fluoroolefins form azeotrope compositions with HF. Generally, the fluoroolefin/HF azeotrope composition will boil at a lower temperature than either of the corresponding pure compounds. Several examples of such fluoroolefin/HF azeotropes are disclosed in U.S. Patent Publication numbers 2007-0100173 A1, 2007-0100174 A1, 2007-0099811 A1, 2007-0100175 A1, 2007-0100176 A1, and 2006-0116538 A1.

It has been unexpectedly calculated that in a few cases azeotrope compositions comprising fluoroolefins and HF may form two liquid phases when condensed and/or cooled. The two phases comprise a fluoroolefinrich phase and an HF-rich phase. This phase behavior allows unique separation schemes utilizing liquid-liquid separation (such as decantation) of the two phases that are not possible with many saturated hydrofluorocarbons, which in general do not phase separate in the same manner.

In one embodiment, the present disclosure provides a process for separating a mixture comprising HF and HFC-1243zf, said process comprising a) feeding the composition comprising HF and HFC-1243zf to a first distillation column; b) removing an azeotrope composition comprising HF and HFC-1243zf as defined above as a first distillate and either i) HF or ii) HFC-1243zf as a first column bottoms composition; c) condensing the first distillate to form two liquid phases, being i) an HF-rich phase and ii) a HFC-1243zf-rich phase; and d) recycling a first liquid phase enriched in the same compound that is removed as the first column bottoms, said first liquid phase being either i) HF-rich phase or ii) HFC-1243zf-rich phase, back to the first distillation column.

Additionally, in another embodiment, the process as described in the paragraph above may further comprise feeding a second liquid phase not recycled in step (d), said second liquid phase being either i) HF-rich phase or ii) HFC-1243zf-rich phase, to a second distillation zone, and recovering the compound not recovered in step (b) as the first column bottoms composition as the second column bottoms composition.

In another embodiment, a process is provided for separating a HFC-1243zf from a mixture comprising hydrogen fluoride and said HFC-1243zf, wherein said HFC-1243zf is present in a concentration greater than the azeotrope concentration for hydrogen fluoride and said HFC-1243zf, said process comprising: a) feeding said mixture comprising hydrogen fluoride and said HFC-1243zf to a first distillation column; b) removing an azeotrope composition comprising hydrogen fluoride and HFC-1243zf as defined above as a first distillate from the first distillation column; c) recovering HFC-1243zf essentially free of hydrogen fluoride as a first bottoms composition from the first distillation column; and d) condensing the first distillate to form two liquid phases, being i) a hydrogen fluoride-rich phase and ii) a HFC-1243zf-rich phase; and e) recycling the HFC-1243zf-rich phase to the first distillation column.

In another embodiment, the process may further comprise:
a) feeding the hydrogen fluoride-rich phase to a second distillation column, and b) recovering hydrogen fluoride essentially free of HFC-1243zf from the bottom of the second distillation column.

In another embodiment, the second distillate comprising HF and HFC-1243zf may be recycled to the two liquid phases.

In one embodiment, wherein the composition comprising HF and HFC-1243zf has a concentration of HFC-1243zf that is greater than the azeotrope concentration for HFC-1243zf and HF, the first distillation column removes the excess HFC-1243zf from the bottom of the column and the azeotrope composition exits the top of the column as the distillate. In another embodiment, the azeotrope composition comprising HF and HFC-1243zf may be condensed and cooled thereby forming two liquid phases, an HF-rich phase and a HFC-1243zf-rich phase.

In one embodiment, the HFC-1243zf-rich phase is recycled back to the first distillation column and the HF-rich phase is fed to a second distillation column. As the HF-rich phase may have HF in excess of the azeotrope composition for HF/HFC-1243zf, the excess HF will be removed from the second distillation column bottom.

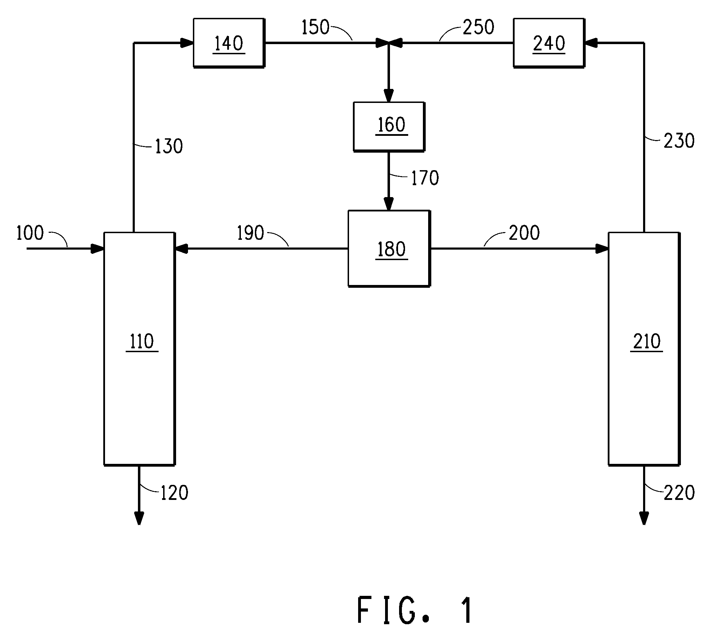

Referring now to FIG 1, one embodiment of this process is illustrated. A composition comprising HF and HFC-1243zf is fed to a first column 110 via stream 100. This first column is operated under appropriate conditions to approach the low-boiling HF/HFC-1243zf azeotrope. Because HFC-1243zf is being fed to this first column in excess of that needed to form the azeotrope with the HF, HFC-1243zf is recovered as the bottoms of the column via stream 120, while a composition near to the HF/HFC-1243zf azeotrope is recovered as distillate via stream 130. Stream 130 is condensed in 140, mixed with a nearly azeotropic composition recycled from a second column 210 via stream 250 and the combined stream is sub-cooled in cooler 160 and sent to decanter 180 where the combined stream 170 separates into separate HFC-1243zf-rich (190) and HF-rich (200) streams. Stream 190 is recycled to the first column as reflux. Stream 200 is fed to the top stage of the second distillation column 210, operated under conditions to approach the HF/HFC-1243zf azeotrope. Because the HF is being fed to this second column in excess of that needed to form the low-boiling HF/HFC-1243zf azeotrope, HF is recovered as the bottoms of the column via stream 220 while a composition close to the HF/HFC-1243zf azeotrope is recovered as distillate via stream 230. Stream 230 is condensed in 240, mixed with the nearly azeotropic composition from the first column via stream 150 and fed to cooler 160 and then decanter 180.

In another embodiment, a process is provided for separating hydrogen fluoride from a mixture comprising hydrogen fluoride and a HFC-1243zf, wherein hydrogen fluoride is present in a concentration greater than the azeotrope concentration for hydrogen fluoride and said HFC-1243zf, said process comprising: a) feeding said mixture comprising hydrogen fluoride and HFC-1243zf to a first distillation column; b) removing an azeotrope composition comprising HFC-1243zf and HF as a first distillate from the first distillation column; c) recovering hydrogen fluoride essentially free of HFC-1243zf from the bottom of the first distillation column; d) condensing the first distillate to form two liquid phases, being an HFC-1243zf-rich phase and a hydrogen fluoride-rich phase; and e) recycling the HF-rich phase to the first distillation column.

In another embodiment, the process may further comprise: a) feeding the HFC-1243zf-rich phase to a second distillation column; and b) recovering HFC-1243zf essentially free of hydrogen fluoride from the bottom of the second distillation column.

In another embodiment, the process may further comprise: recycling the hydrogen fluoride-rich phase to the first distillation column.

In another embodiment, the composition comprising HF and HFC-1243zf has a greater concentration of HF than the azeotrope composition for HF and HFC-1243zf. The excess HF may be removed from the bottom of the first distillation column and the azeotrope composition exits as the distillate. In another embodiment, the azeotrope composition comprising HF and HFC-1243zf may be condensed and cooled thereby forming two liquid phases, an HF-rich phase and a HFC-1243zf-rich phase. For this embodiment, the HF-rich phase is recycled back to the first distillation column and the HFC-1243zf-rich phase is fed to a second distillation column. As the HFC-1243zf-rich phase may have HFC-1243zf in excess of the azeotrope composition for HF/HFC-1243zf, the excess HFC-1243zf may be removed from the second distillation column bottom as HFC-1243zf essentially free of HF.

Referring again to FIG 1, another embodiment of this process is illustrated. A composition comprising HF and HFC-1243zf is fed to a first column 110 via stream 100. This first column is operated under appropriate conditions to approach the low-boiling HF/HFC-1243zf azeotrope. Because HF is being fed to this first column in excess of that needed to form the azeotrope with the HFC-1243zf, HF is recovered as the bottoms of the column via stream 120, while a composition near to the HF/HFC-1243zf azeotrope is recovered as distillate via stream 130. Stream 130 is condensed in 140, mixed with a nearly azeotropic composition recycled from a second column via stream 250 and the combined stream is sub-cooled in cooler 160 and sent to decanter 180 where the combined stream 170 separates into separate HF-rich (190) and HFC-1243zf-rich (200) streams. Stream 190 is recycled to the first column as reflux. Stream 200 is fed to the top stage of the second distillation column 210, operated under conditions to approach the HF/HFC-1243zf azeotrope. Because HFC-1243zf is being fed to this second column in excess of that needed to form the low-boiling HF/HFC-1243zf azeotrope, HFC-1243zf is recovered as the bottoms of the column via stream 220, while a composition close to the HF/HFC-1243zf azeotrope is recovered as distillate via stream 230. Stream 230 is condensed in 240, mixed with the nearly azeotropic composition from the first column via stream 150 and fed to cooler 160 and then decanter 180.

In one embodiment the operating conditions for the first and second distillation columns will depend upon the HFC-1243zf being purified and the relative amounts of HF and HFC-1243zf in the composition to be separated.

In one embodiment, the first and second distillation column may operate at from about 14.7 psia (101 kPa) to about 300 psia (2068 kPa), with a top temperature of from about -50°C to about 200°C and a bottom temperature from about -30°C to about 220°C. In another embodiment, the pressure will range from about 50 psia (345 kPa) to about 250 psia (1724 kPa), with a top temperature of from about -25°C to about 100°C and a bottom temperature from about 0°C to about 150°C.

### 4. Separation process - Azeotropic distillation with an entrainer (not part of the invention)

Azeotropic distillation for separating HFC-1243zf from mixtures of HF and HFC-1243zf may, in another embodiment, be carried out using an entrainer compound. For the process including an entrainer, the azeotrope composition need not phase separate upon condensing and cooling as described above.

In one embodiment, the entrainer serves to provide an improved liquid-liquid phase separation for a system wherein that separation would otherwise not be effective.

In one embodiment, the HFC-1243zf is present in the HF/HFC-1243zf mixture in a concentration greater than the azeotrope concentration for said HFC-1243zf and HF. Thus, in one embodiment is provided a process for the purification of a HFC-1243zf from a mixture comprising HFC-1243zf and HF, wherein said HFC-1243zf is present in said mixture in a concentration greater than the azeotrope concentration for said HFC-1243zf and HF, said process comprising:
a. adding an entrainer to the mixture comprising HFC-1243zf and HF thus forming a second mixture;
b. distilling said second mixture in a first distillation step to form a first distillate composition comprising HF, HFC-1243zf, and entrainer, and a first bottoms composition comprising HFC-1243zf essentially free of HF and entrainer;
c. condensing said first distillate composition to form two liquid phases, being i) an HF-rich phase and ii) an entrainer-rich phase; and
d. optionally recycling the entrainer-rich phase back to the first distillation step. In another embodiment, the process further comprises feeding the HF-rich phase to a second distillation step and forming a second distillate composition comprising entrainer, HFC-1243zf and HF and a bottoms composition comprising HF essentially free of HFC-1243zf and entrainer. In another embodiment, the process may further comprise recycling said second distillate composition back to the two liquid phases.

The process for separating HFC-1243zf from a first composition comprising HF and HFC-1243zf comprises contacting said first composition with an entrainer to form a second composition. The contacting may occur in a first distillation column, or the second composition may be formed by mixing the components prior to feeding to a distillation column in a pre-mixing step.

The weight ratio of the HF and HFC-1243zf in the first composition will depend upon the means of producing the composition. In one embodiment, the HF may be from about 3 weight percent to about 85 weight percent of the composition; the HFC-1243zf may be from about 97 weight percent to about 15 weight percent.

In another embodiment, the HF may be from about 5 weight percent to about 50 weight percent and the HFC-1243zf may be from about 95 weight percent to about 50 weight percent

In yet another embodiment the composition comprising HF and HFC-1243zf may be produced in a dehydrofluorination reactor resulting in a 50/50 mole ratio of HF to the HFC-1243zf.

In one embodiment, the compositions comprising HF and HFC-1243zf may be prepared by any convenient method to combine the desired amounts of the individual components. A preferred method is to weigh the desired component amounts and thereafter combine the components in an appropriate vessel. Agitation may be used, if desired.

Alternatively, the compositions comprising HF and HFC-1243zf may be prepared by feeding the effluent from a reactor, including a dehydrofluorination reactor that contains HF and HFC-1243zf, to the first distillation column. The entrainer may be added at a separate feed point such that the second composition is formed directly in the distillation column. Alternatively, the entrainer may be mixed with the first composition comprising HF and HFC-1243zf thus forming the second composition prior to the distillation column in a pre-mixing step.

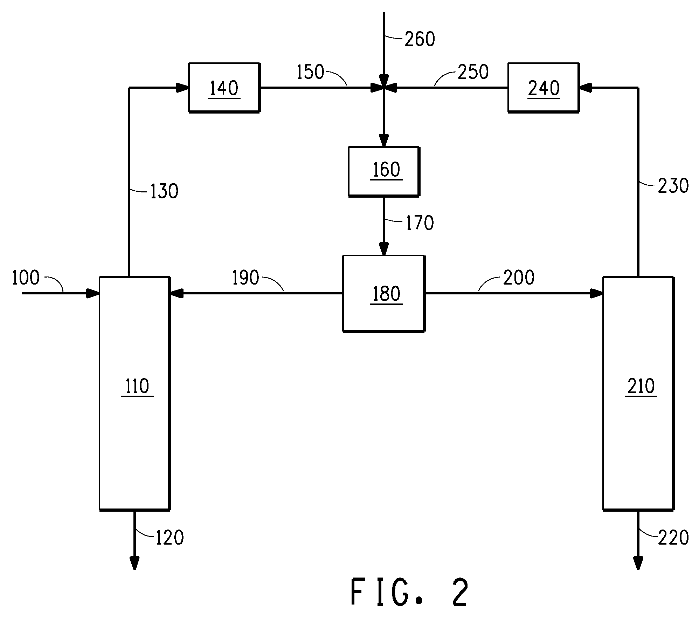

In one embodiment of the separation process, a composition comprising HFC-1243zf and HF is fed directly to a first distillation column. In another embodiment, the HFC-1243zf and HF may be pre-mixed with an entrainer prior to the distillation column. The pre-mixing step may occur in a cooler (160 in FIG 2). Then the cooled mixture is fed to a decanter (180 in FIG 2) prior to feeding to the distillation column.

In one embodiment, the first distillate composition comprises a low boiling azeotrope of HF and entrainer optionally containing minor amounts of HFC-1243zf. Further, in another embodiment, the HFC-1243zf essentially free of HF and optionally minor amounts of entrainer may be recovered from the bottom of the first distillation column.

The operating variables for the first distillation column will depend strongly on the entrainer being used in the separation process. In general the first distillation column may operate at pressures from about 14.7 psia (101 kPa) to about 500 psia (3448 kPa) with a top temperature of from about -50°C to about 100°C and a bottom temperature of from about -30 °C to about 200°C. In another embodiment, the first distillation column will operate at pressures from about 100 psia (690 kPa) to about 400 psia (2758 kPa) with a top temperature of from about - 50°C to about 50°C and a bottom temperature from about 10°C to about 150°C.

It was surprisingly calculated that in some few cases, azeotropes of HF and compounds used as entrainers will separate into HF-rich and entrainer-rich liquid fractions upon condensing and being cooled. In one embodiment, the first distillate composition may be fed to a liquid separation zone (e.g. decanter). The first distillate composition comprising an azeotrope of HF and entrainer may be phase separated forming two liquid phases, one being HF-rich and the other being entrainer-rich. The lower density phase may be recovered from the top of the liquid separation zone and the higher density phase may be recovered from the bottom of the liquid separation zone. The entrainer-rich phase (whether higher or lower density) may be fed back to the first distillation column. In one embodiment the HF-rich phase may be fed to a second distillation column or in another embodiment, the HF-rich phase may be split to send some portion back to the first distillation column (in order to provide more reflux and allow the first distillation column to operate properly) and the remainder may be fed to the second distillation column. The second distillation column allows recovery of HF essentially free of HFC-1243zf and entrainer as a bottoms composition. The top composition comprising HFC-1243zf, HF and entrainer may be recycled to the liquid separation zone, be utilized in some other manner, or disposed. The operating variables for the second distillation column will depend strongly on the entrainer being used in the separation process. In general the second distillation column may operate at pressures from about 14.7 psia (101 kPa) to about 500 psia (3448 kPa) with a top temperature of from about - 50°C to about 100°C and a bottom temperature of from about -30°C to about 200°C. In another embodiment, the first distillation column will operate at pressures from about 100 psia (690 kPa) to about 400 psia (2758 kPa) with a top temperature of from about -25°C to about 50°C and a bottom temperature from about zero °C to about 150°C.

Referring now to FIG 2, a composition comprising HF and HFC-1243zf is fed to a first distillation column 110 via stream 100. An entrainer-rich composition is also fed to the top stage of column 110 via stream 190. If the combined amount of HFC-1243zf in streams 100 and 190 is in excess of that needed to form the low-boiling HF/HFC-1243zf azeotrope, HFC-1243zf is recovered essentially free of both HF and entrainer from the bottom of column 110 via stream 120. A ternary composition comprising HF, HFC-1243zf, and entrainer, but enriched in HFC-1243zf relative to stream 190, leaves the top of the first column as the first distillate stream 130. Stream 130 is condensed by condenser 140 forming stream 150 and mixed with a condensed second distillate stream 250 from a second distillation column. In one embodiment, additional entrainer may be added via stream 260, if needed. Combined streams 150, 250, and 260 are fed to cooler 160 and then to decanter 180 where the sub-cooled liquid stream 170 separates into entrainer-rich and HF-rich liquid phase compositions which leave the decanter via streams 190 and 200, respectively. The HFC-1243zf present distributes between the two liquid phases with the majority ending up in the entrainer-rich phase. The HF-rich composition stream 200 is fed to the top stage of the second distillation column 210. Because the amount of HF in stream 200 is in excess of that needed to form a low-boiling HF/HFC-1243zf azeotrope, HF is recovered as a product stream essentially free of both HFC-1243zf and entrainer from the bottom of column 210 via stream 220. A ternary composition comprising HF, HFC-1243zf and entrainer, but enriched in entrainer relative to stream 200, leaves the top of the second column as the second distillate stream 230. Stream 230 is condensed in condenser 240, forming stream 250, and combined with streams 150 and 260 previously described.

Alternatively, in another embodiment, rather than feed the HF/HFC-1243zf mixture directly to the distillation column 110, the mixture may be fed to cooler 160 and then to decanter 180 where the mixture phase separates. Then stream 190 carries the mixture of HF, HFC-1243zf and entrainer to the first distillation column 110.

In another embodiment, the concentration of HF in the HF/HFC-1243zf mixture is greater than the concentration in the azeotrope of HFC-1243zf and HF. Thus, in another embodiment is provided a process for the purification of HF from a mixture comprising a HFC-1243zf and HF, wherein HF is present in a concentration greater than the azeotrope concentration for HF and said HFC-1243zf, said process comprising:
a. adding an entrainer to the mixture comprising HFC-1243zf and HF thus forming a second mixture;
b. distilling said second mixture in a first distillation step to form a first distillate composition comprising HF, entrainer, and a HFC-1243zf, and a first bottoms composition comprising HF essentially free of HFC-1243zf and entrainer;
c. condensing said first distillate composition to form two liquid phases, being i) an entrainer-rich phase and ii) an HF-rich phase; and
d. optionally recycling the HF-rich phase back to the first distillation step. In another embodiment, the process may further comprising feeding the HF-rich phase to a second distillation step and forming a second distillate composition comprising entrainer, HF, and HFC-1243zf, and a bottoms composition comprising HFC-1243zf essentially free of entrainer. In another embodiment, the process may further comprise recycling said second distillate composition back to the two liquid phases.

Referring again to FIG 2, a composition comprising HF and HFC-1243zf is fed to a first distillation column 110 via stream 100. An HF-rich composition is also fed to the top stage of column 110 via stream 190. If the combined amount of HF in streams 100 and 190 is in excess of that needed to form the low-boiling HF/HFC-1243zf azeotrope, HF is recovered essentially free of both HFC-1243zf and entrainer from the bottom of column 110 via stream 120. A composition near the HF/HFC-1243zf azeotrope with a minor amount of entrainer is recovered as the first distillate via stream 130. Stream 130 is condensed by condenser 140 forming stream 150 and mixed with a condensed second distillate stream 250 from a second distillation column. In one embodiment, additional entrainer may be added via stream 260, if needed. Combined streams 150, 250, and 260 are fed to cooler 160 and then to decanter 180 where the sub-cooled liquid stream 170 separates into HF-rich and entrainer-rich liquid phase compositions which leave the decanter via streams 190 and 200, respectively. The HFC-1243zf present distributes between the two liquid phases with the majority ending up in the entrainer-rich phase. The entrainer-rich composition stream 200 is fed to the top stage of the second distillation column 210. Because the amount of HFC-1243zf in stream 200 is in excess of that needed to form a low-boiling entrainer/HFC-1243zf azeotrope, HFC-1243zf is recovered as a product stream essentially free of both HF and entrainer from the bottom of column 210 via stream 220. A ternary composition comprising entrainer, HFC-1243zf, and HF, but enriched in entrainer relative to stream 200 leaves the top of the second column as the second distillate stream 230. Stream 230 is condensed in condenser 240, forming stream 250, and combined with streams 150 and 260 previously described.

Alternatively, in another embodiment, rather than feed the HF/HFC-1243zf mixture directly to the distillation column 110, the mixture may be fed to cooler 160 and then to decanter 180 where the mixture phase separates. Then stream 190 carries the mixture of HF, HFC-1243zf and entrainer as the HF-rich phase to the first distillation column 110.

### 5. Separation of HFC-254 from HFC-1243zf HF

HFC-1243zf may be produced by dehydrofluorination of certain HFC-254 (tetrafluoropropane) isomers. By HFC-254 is meant any isomer of tetrafluoropropane and any combinations of any isomers of tetrafluoropropane that can yield HFC-1243zf upon dehydrofluorination. Isomers of tetrafluoropropane include HFC-254eb (1,1,1,2-tetrafluoropropane) and HFC-254fb (1,1,1,3-tetrafluoropropane).

In one embodiment, a process is provided for the separation of HFC-1243zf from a mixture of HFC-1243zf, HF, and at least one of HFC-254eb or HFC-254fb, said process comprising:
a) subjecting said mixture to a first distillation step, wherein additional HFC-1243zf is fed from a second distillation step, to form a first distillate comprising an azeotrope of HFC-1243zf and HF and a first bottoms composition comprising at least one of HFC-254eb or HFC-254fb;
b) feeding said first distillate to a second distillation step to form a second distillate comprising an azeotrope of HFC-1243zf and HF and a second bottoms composition comprising HFC-1243zf essentially free of HF;
c) condensing said second distillate to form two liquid phases, being i) an HF-rich phase and ii) an HFC-1243zf-rich phase; and
d) recycling the HFC-1243zf-rich phase from (c) back to the second distillation step. In another embodiment, the process may further comprise feeding the HF-rich phase to a third distillation step to form a third distillate comprising an azeotrope of HFC-1243zf and HF and a third bottoms composition comprising HF essentially free of HFC-1243zf.

In this embodiment the azeotropic distillation involves providing an excess of HFC-1243zf to the distillation column in addition to that produced from the dehydrofluorination reaction of HFC-254eb and/or HFC-254fb. In this embodiment, HFC-1243zf serves as an entrainer in the distillation process. If the proper total amount of HFC-1243zf is fed to the column, then all the HF may be taken overhead as an azeotrope composition containing HFC-1243zf and HF. Enough HFC-1243zf can be provided, for example, by feeding supplemental HFC-1243zf to the distillation column over that exiting in the dehydrofluorination reaction product stream. Thus, the HFC-254eb and/or HFC-254fb removed from the column bottoms may be essentially free of HF.

For example, a reactor product mixture comprising HF, HFC-1243zf and HFC-254eb may be fed to a first distillation column operated under conditions to form the HF/ HFC-1243zf azeotrope with the HF/ HFC-1243zf azeotrope being removed from the distillation column as the overhead distillate. The HF in this distillate may then be separated and removed from the HFC-1243zf by other means, e.g. by using pressure swing distillation or the methods as disclosed herein. Some portion of the HFC-1243zf so obtained can be recycled back to the first distillation column in quantities sufficient so that all the HF fed to the first distillation column is removed from that column as the HF/ HFC-1243zf azeotrope, thus producing a HFC-254eb bottoms stream essentially free of HF.

Where the composition to be separated is formed by dehydrohalogenating either of HFC-254eb or HFC-254fb, it is desirable to recycle any unreacted HFC-254eb or HFC-254fb back to the reactor so that they may be converted to HFC-1243zf. However, it is necessary that HF and HFC-1243zf be removed from said unreacted HFC-254eb or HFC-254fb prior to being recycled so as not to inhibit the equilibrium reaction. It is also necessary that the HF be removed from the HFC-1243zf to allow its use as a refrigerant or in other applications.

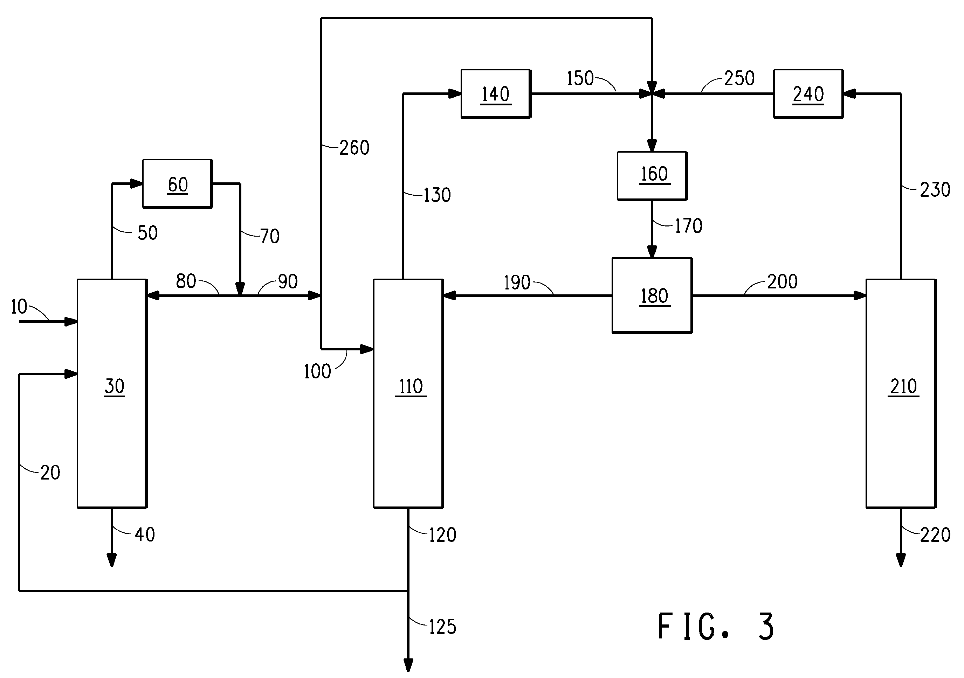

Referring now to FIG 3, a stream comprising HF, HFC-1243zf, and at least one of HFC-254eb and HFC-254fb is fed to a first distillation column via stream 10, with the column operated under conditions to approach the low-boiling HF/HFC-1243zf azeotrope, which is removed as distillate via streams 50, 70, and 90. Enough supplemental HFC-1243zf is recycled from the second column bottoms to this first column via stream 20 to enable all of the HF to be removed from the HFC-254fb and/or HFC-254eb. The HFC-254fb and/or HFC-254eb are obtained essentially free of HFC-1243zf and HF as the bottoms product from this column via stream 40.

The near HF/HFC-1243zf azeotropic composition in stream 50 is condensed and divided into reflux 80 and distillate 90 streams. Distillate stream 90 may be fed to a second distillation column 110 via stream 100 as shown and indicated, mixed with distillate streams 150 and 250 from the second and third columns, respectively, and sent to cooler 160 and decanter 180, or be divided between these two destinations. Because of the desire to remove all of the HF overhead in column 30, excess HFC-1243zf would be recycled to column 30, making the composition of streams 50, 70, 80, 90, and 100 lie on the HFC-1243zf-rich side of the azeotrope. Therefore, if distillate stream 90 is sent via stream 100 to a second distillation column, it should be sent to the column which produces purified HFC-1243zf as the bottoms product.

In one embodiment, distillate stream 90 via stream 260 is mixed with distillate streams 150 and 250 from the second and third columns, respectively, and sent to cooler 160, forming sub-cooled stream 170, which is fed to decanter 180. In the decanter, stream 170 separates into HFC-1243zf-rich and HF-rich liquid fractions, which are removed as streams 190 and 200. The HFC-1243zf-rich stream from the decanter is fed via stream 190 to a second distillation column 110 containing 19 theoretical stages and operated under conditions to approach the HFC-1243zf/ HF azeotrope, which is distilled overhead as distillate stream 130, condensed in condenser 140, and mixed with the distillates from the first and third columns via stream 150. Column 110 produces a bottoms stream of HFC-1243zf essentially free of HF via stream 120. Part of the HFC-1243zf bottoms stream 120 is recycled to the first column via stream 20, as previously described, and the rest becomes the purified HFC-1243zf product removed via stream 125. The HF-rich stream from the decanter is fed via stream 200 to a third distillation column 210 operated under conditions to approach the HFC-1243zf/HF azeotrope, which is distilled overhead as distillate as stream 230 which is condensed in condenser 250 and mixed with the distillates from the first and second columns via stream 250. Column 210 produces a bottoms stream of HF essentially free of HFC-1243zf via stream 220.

In another aspect of this invention, an entrainer may be added to enable separation of the HF from the HFC-1243zf, or of the HF from the HFC-1243zf and HFC-254eb and/or HFC-254fb.

For example, the mixture of HF, HFC-1243zf, HFC-254eb and/or HFC-254fb may be formed by any practical means, such as by feeding at least one of HFC-254fb or HFC-254eb over a chrome oxide catalyst at elevated temperature. The mixture of HF, HFC-1243zf, HFC-254eb and/or HFC-254fb may be fed to a distillation column. A suitable entrainer is then also fed to the distillation column, either as a separate stream or by being mixed in with the HF/HFC-1243zf/HFC-254fb and/or HFC-254eb mixture prior to feeding it to the distillation column. The distillation column is then operated under conditions sufficient to form a low-boiling azeotrope composition between the entrainer and HF, with the HF and entrainer removed as the column distillate, and the HFC-1243zf, HFC-254eb and/or HFC-254fb recovered from the column bottoms essentially free of HF. The HFC-1243zf may then be separated from the HFC-254eb and/or HFC-254fb by any usual means including conventional distillation, with the HFC-1243zf being recovered as product and with the HFC-254eb and/or HFC-254fb optionally being recycled back to the reaction step to produce HFC-1243zf.

Thus in another embodiment is provided a process for separating HF from a mixture comprising HFC-1243zf, HF, and at least one of HFC-254eb or HFC-254fb. The process comprises:
a. adding an entrainer to the mixture comprising HFC-1243zf, HF, and at least one of HFC-254eb or HFC-254fb thus forming a second mixture;
b. distilling said second mixture in a first distillation step to form a first distillate composition comprising HF and entrainer and a first bottoms composition comprising HFC-1243zf and at least one of HFC-254eb or HFC-254fb;
c. condensing said first distillate composition to form two liquid phases, being (i) an entrainer-rich phase and (ii) an HF-rich phase; and
d. recycling the entrainer-rich phase back to the first distillation step.

In another embodiment, the process may further comprise feeding the HF-rich phase to a second distillation step and forming a second distillate composition comprising an azeotrope of entrainer and HF and a second bottoms composition comprising HF essentially free of entrainer. In another embodiment, the process may further comprise recycling said second distillate composition back to the two liquid phases.

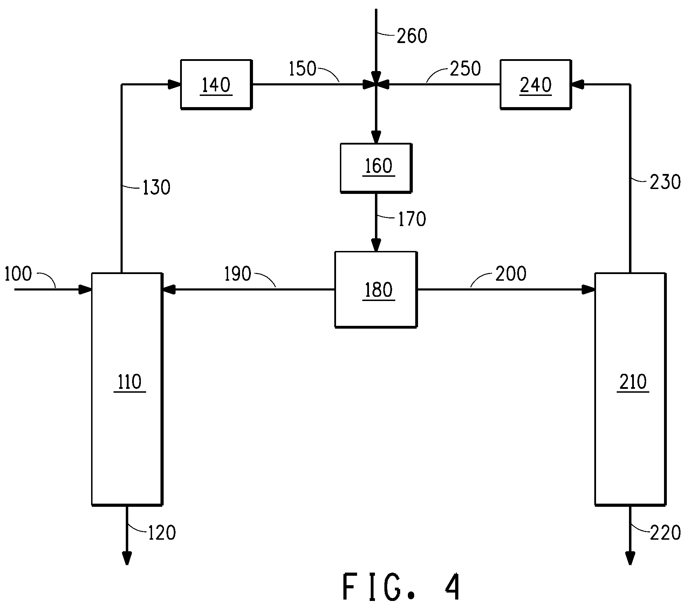

Referring now to FIG 4, a stream comprising HF, HFC-1243zf, and at least one of HFC-254eb or HFC-254fb is fed to a first distillation column 110 via stream 100. An entrainer-rich stream is also fed to this column via stream 190. Column 110 is operated under conditions to cause HF to distill overhead with the entrainer due to the influence of the low-boiling HF/entrainer azeotrope. Sufficient entrainer is fed to this first column via stream 190 such that HFC-1243zf and HFC-254eb or HFC-254fb may be obtained essentially free of entrainer and HF as the bottoms from column 110 via stream 120. The HFC-1243zf and HFC-254eb or HFC-254fb in stream 120 may then optionally be separated from each other by conventional distillation and the HFC-254eb or HFC-254fb optionally recycled back to a dehydrofluorination reactor to form HFC-1243zf. The distillate from column 110, removed via stream 130, contains essentially all of the entrainer and HF in column feeds 100 and 190 and, optionally, some HFC-254eb or HFC-254fb and/or HFC-1243zf. This first distillate stream 130 is condensed by condenser 140 to form stream 150, which is then mixed with condensed distillate stream 250 from the second distillation column and, as needed, additional fresh entrainer added via stream 260. This combined stream is sub-cooled by cooler 160 and sent via stream 170 to decanter 180 where it separates into separate entrainer-rich and HF-rich liquid fractions which are removed via streams 190 and 200, respectively. The majority of the HFC-254eb or HFC-254fb and HFC-1243zf present in the decanter partition into the entrainer-rich phase fraction. The entrainer-rich fraction is fed to the first distillation column 110 via stream 190. The HF-rich fraction from the decanter is fed via stream 200 to a second distillation column 210 containing 8 theoretical stages and operated under conditions such that a bottoms stream of HF essentially free of HFC-254eb or HFC-254fb, HFC-1243zf, and entrainer is produced and removed via stream 220. The distillate from column 210, removed via stream 230 and containing essentially all of the HFC-254eb or HFC-254fb, HFC-1243zf, and entrainer present in the column feed (stream 200) plus the HF not recovered in product stream 220, is condensed by condenser 240 and removed via stream 250. Condensed distillate stream 250 is combined with both the condensed distillate stream 150 from the first column and, as needed, fresh entrainer, added via stream 260, then cooled and fed to the decanter for further separation.

In another embodiment, a hydrofluorocarbon (HFC), which forms a homogeneous azeotrope with HF, can be separated from a mixture comprising HF, the HFC and a HFC-1243zf by azeotropic distillation using the HFC-1243zf as an entrainer, followed by separation of the HFC-1243zf and HF by azeotropic distillation using an added compound as the entrainer. HF and the HFC-1243zf are not required to be partially miscible at reduced temperatures for such a separation process to work as long as the HF-HFC-1243zf azeotrope has a lower boiling point than the HF-HFC azeotrope. For illustration purposes, the HFC-1243zf is HFC-1243zf and the HFC is HFC-254eb and/or HFC-254fb.

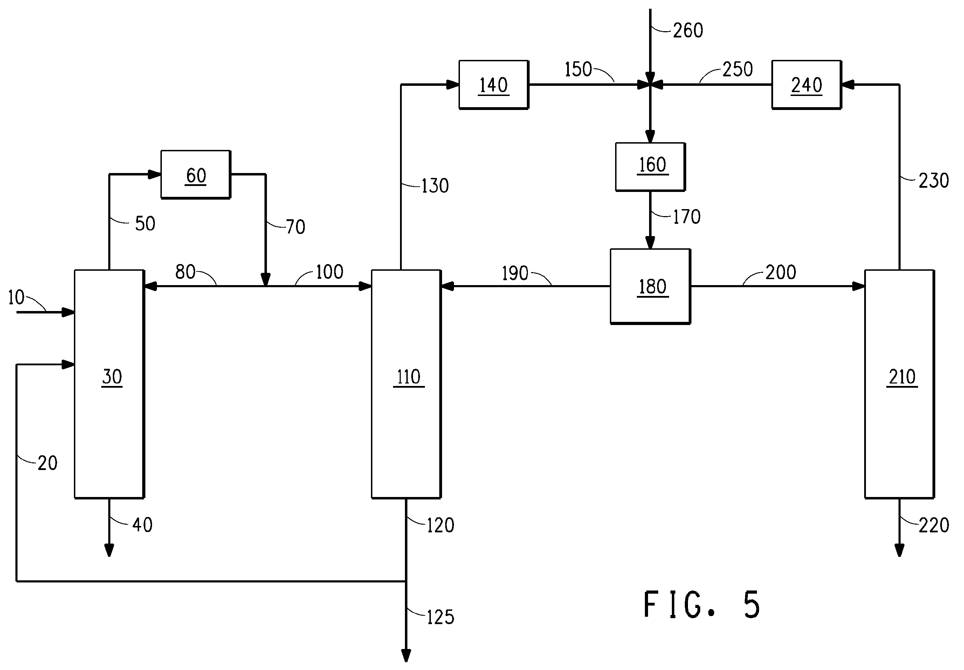

Referring now to FIG 5, a stream comprising HF, HFC-1243zf, and at least one of HFC-254eb and HFC-254fb is fed to a first distillation column 30 via stream 10, with the column operated under conditions to approach the low-boiling HF/HFC-1243zf azeotrope, which is removed from the top of the column via stream 50, condensed in condenser 60, and the condensed stream 70 is split into reflux 80 for column 30 and distillate 100 that is fed to column 110. This first column 30 can be designed and operated in such a way that the near azeotropic distillate is essentially free of HFC-254eb and/or HFC-254fb. In one embodiment, the amount of HFC-1243zf in feed stream 10 may be insufficient to form a near azeotropic mixture with all of the HF in stream 10. However, for this embodiment, by recycling enough supplemental HFC-1243zf from the second column bottoms to the first column via stream 20, essentially all of the HF can be distilled overhead as the HF/HFC-1243zf azeotrope such that HFC-254fb and/or HFC-254eb are obtained essentially free of HFC-1243zf and HF as the bottoms product from column 30 via stream 40. The HFC-254eb and/or HFC-254fb may then optionally be recycled back to a reactor for production of HFC-1243zf, or may optionally be further purified and then recycled. This demonstrates the use of the HFC-1243zf as an entrainer to remove HF from an HFC.

As described for FIG 3, in one embodiment, the distillate 50 from the first column 30 may be fed to a second distillation column 110. In another embodiment, the distillate 50 may be mixed with the distillate streams 130 and 230 from a second column 110 and a third column 210, respectively, and the mixed streams cooled in cooler 160, and then sent to a decanter 180. In yet another embodiment, the distillate 50 may be split between the second distillation column 110 and a stream to be mixed with the other distillate streams 130 and 230.

In the embodiment illustrated in FIG 5, the distillate from the first column 30 is fed via stream 100 to the top stage of the second column 110. An entrainer-rich stream is also fed to this second column 110 via stream 190. Distillation column 110 is operated under conditions such that the distillate, removed via stream 130, contains essentially all of the entrainer and HF in the column feeds 100 and 190 and produces an HFC-1243zf bottoms product essentially free of HF and entrainer which is removed via stream 120. Part of the HFC-1243zf bottoms stream 120 is recycled to the first column via stream 20, as previously described, and the rest becomes the purified HFC-1243zf product removed via stream 125. Distillate stream 130 is condensed by condenser 140 to form stream 150, which is then mixed with the condensed distillate stream 250 from the second distillation column and, as needed, fresh entrainer added via stream 260. This combined stream is cooled by cooler 160 and sent via stream 170 to decanter 180 where it separates into entrainer-rich and HF-rich liquid phase fractions, which are removed via streams 190 and 200, respectively. The majority of the HFC-1243zf present in the decanter partitions into the entrainer-rich phase fraction. The decanter entrainer-rich fraction is fed to column 110 via stream 190. The decanter HF-rich fraction is fed, via stream 200, to a third distillation column 210 operated under conditions which produce a bottoms product consisting of HF essentially free of HFC-1243zf and the entrainer, which is removed via stream 220. The distillate from column 210, which is removed via stream 230 and contains essentially all of the HFC-1243zf and entrainer present in the column feed (stream 200) and any HF not recovered in product stream 220, is condensed by condenser 240, forming stream 250. Condensed distillate stream 250 is combined with both the condensed distillate stream 150 from the second column and, as needed, fresh entrainer, added via stream 260, then cooled and fed to the decanter via stream 170 for further separation.

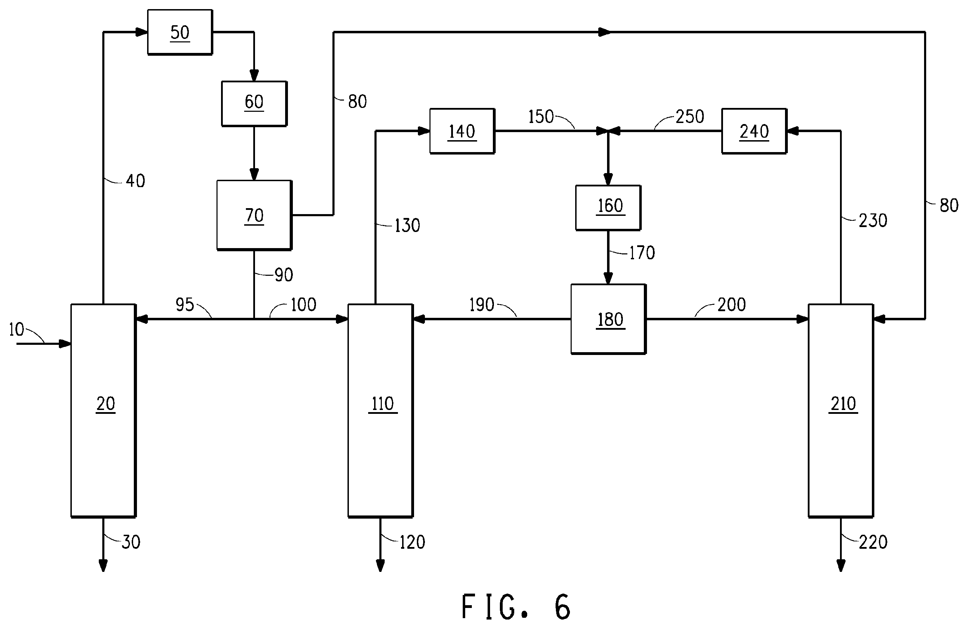

In another embodiment, as illustrated by FIG 6, HFC-254eb and/or HFC-254fb and HFC-1243zf can both be separated from a mixture comprising HF, HFC-254eb and/or HFC-254fb and HFC-1243zf by azeotropic distillation using the HFC-1243zf as an entrainer.

Referring now to FIG 6, the difference in this figure when compared to FIG 5, is that a first cooler 60 and a first decanter 70 are added after the first distillation column's condenser 50 such that the distillate separates into HF-rich and HFC-1243zf-rich liquid phase fractions in the decanter, which are removed via streams 80 and 90, respectively. Part of the HFC-1243zf-rich stream 90 is returned to the first column as reflux via stream 95 and the remaining portion is fed to a second distillation column 110 via stream 100. Column 210 separates stream 100 into an HFC-1243zf bottoms product, removed via stream 120 that is essentially free of HF and a distillate composition near to the HF/HFC-1243zf azeotrope, removed via stream 130, as illustrated in FIG 5. Because the reflux stream 95 is enriched in HFC-1243zf relative to the HFC-1243zf/HF azeotropic composition, the reflux stream 95 supplies the additional HFC-1243zf needed to make the HFC-254eb and/or HFC-254fb bottoms product from the first column, removed via stream 30, essentially free of HF, thereby reducing the amount of purified HFC-1243zf that must be recycled from the second column to the first column. As illustrated in FIG 6, at sufficiently high reflux flows, the need for recycling any of the purified HFC-1243zf from the bottom of the second column to the first column can be completely eliminated. In another embodiment, wherein the reflux flow is not high enough to allow all the HF to go overhead with the HFC-1243zf, there may be some recycle from the product stream 120 back to the first column (not shown in any figure), therefore this embodiment would include both the decanter 70 with streams 90 and 95 (as in FIG 6) and a recycle stream from stream 120 back to the first distillation column (as stream 20 feeding column 30 in FIG 5.

The first decanter's HF-rich phase fraction 200 is fed to a third distillation column 210 via stream 80. Both feeds (streams 80 and 200) to the third column have compositions containing excess HF relative to the HF/HFC-1243zf azeotrope so that an HF bottoms product essentially free of HFC-1243zf may be obtained from column 210 and removed via stream 220. The distillate from the third column has a composition near to the HF/HFC-1243zf azeotrope and is removed via stream 230. As in earlier examples, the distillates (streams 130 and 230) from columns 110 and 210 are condensed in condensers 140 and 240, forming streams 150 and 250, respectively, mixed together, and sent first to a second cooler (160) and then to a second decanter (180) where separate HFC-1243zf-rich and HF-rich liquid phase fractions are formed. The HFC-1243zf-rich fraction is removed from decanter 180 via stream 190 and fed to the second column 110 for further separation. The HF-rich fraction is removed from decanter 180 via stream 200 and fed to the third column 210 for further separation.

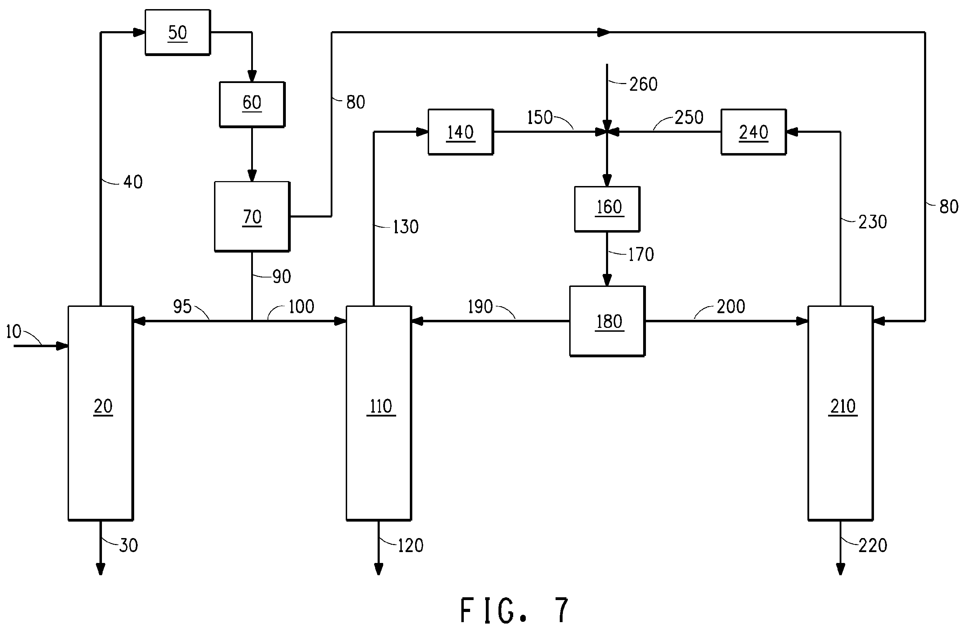

In another embodiment, HFC-254eb and/or HFC-254fb and HFC-1243zf can both be separated from a mixture comprising HF, HFC-254eb and/or HFC-254fb, and HFC-1243zf by azeotropic distillation using an added entrainer, as illustrated in FIG 7.

Referring now to FIG 7, the first distillation column 20, condenser 50, cooler 60, and decanter 70 in this embodiment operates identically to the similarly numbered equipment in FIG 6 as just described. The HF-rich and HFC-1243zf-rich liquid distillate fractions from the first column's decanter 70 are fed via streams 80 and 100 to distillation columns 210 and 110 which recover purified HF and HFC-1243zf, respectively. The remaining portion of the process shown in FIG 7, i.e., distillation columns 110 and 210, condensers 140 and 240, cooler 160, decanter 180, and all of their associated streams, have the same function and operate similarly to the same numbered equipment shown and described for FIG 5.

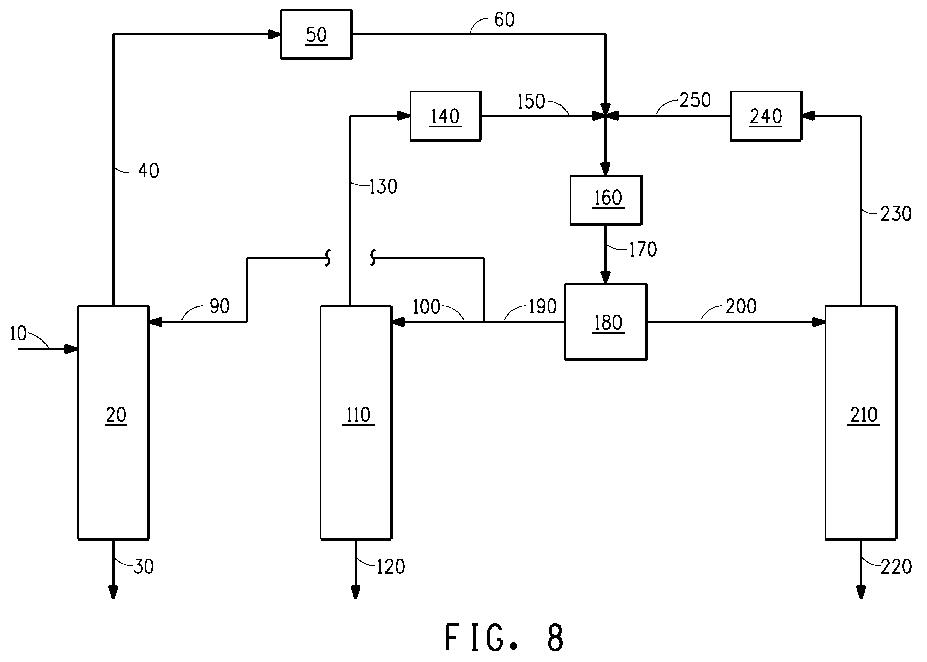

In other embodiments of the invention, certain pieces of equipment can serve multiple distillation columns. For instance, in one embodiment, condensers 140 and 240 may be combined into a single unit, thus streams 130 and 230 of FIG 7 would both feed into the single condenser. In another embodiment, coolers 60 and 160 of FIG 7 can be combined into a single unit, shown as cooler 160 in FIG 8. In another embodiment, decanters 70 and 180 of FIG 7 can be combined into a single unit, as shown by decanter 180 in FIG 8. In yet another embodiment, the three condensers 50, 140 and 240 of FIG 7 can be combined into a single unit, thus streams 40, 130 and 230 of FIG 7 would all feed into the one condenser.

In one embodiment, entrainers for HF separation from HFC-1243zf and optionally HFC-254eb and/or HFC-254fb include: methane, ethane, ethylene, acetylene, vinylacetylene, propane, propylene, propyne, cyclopropane, cyclopropene, propadiene, methyl chloride (CH₃Cl), dichlorodifluoromethane (CFC-12), 2-chloro-1,1,2-trifluoroethylene, chloropentafluoroethane (CFC-115), 2-chloro-1,1,3,3,3-pentafluoropropene (CFC-1215xc), 2-chloro-1,1-difluoroethylene (HCFC-1122), 1,1,2-trifluoroethylene (HFC-1123), 1,1-difluoroethylene (HFC-1132a), 2,3,3,3-tetrafluoropropene (HFC-1234yf), hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), trifluoromethyl-difluoromethyl ether (CF₃OCHF₂, HFOC-125E), 1,1-difluorodimethyl ether, tetrafluorodimethylether (HFOC-134E), difluoromethyl methyl ether (CHF₂OCH₃, HFOC-152aE), pentafluoroethyl methyl ether, HFPO, chlorine (Cl₂), hexafluoroacetone, PMVE (perfluoromethylvinylether), PEVE (perfluoroethylvinylether), and mixtures thereof.

In another embodiment, the entrainer that is effective for separation of HF from HFC-1243zf and optionally HFC-254eb and/or HFC-254fb comprises propane.

### EXAMPLES

The concepts described herein will be further described in the following examples.

### EXAMPLE 1

This example demonstrates the existence of azeotropic or azeotrope-like compositions between the binary pairs consisting essentially of HFC-1243zf and HF. To determine the relative volatility of each binary pair, the PTx Method was used. In this procedure, for each binary pair, the total absolute pressure in a sample cell having a volume of 85 ml was measured at constant temperature for various binary compositions. These measurements were then reduced to equilibrium vapor and liquid compositions using the NRTL equation. The azeotropic compositions measured at about 29.8°C and at about 79.7°C are listed in Table 1 along with calculated values for the azeotrope at other temperatures and pressures.

**TABLE 1**

| Temperature, °C | Pressure, psia (kPa) | Mole % HF | Mole % HFC-1243zf |
|---|---|---|---|
| -20 | 19.9 (137.2) | 32.8 | 67.2 |
| -10 | 29.6 (204.1) | 32.4 | 67.6 |
| 0 | 42.5 (293.0) | 31.2 | 68.8 |
| 10 | 59.2 (408.2) | 30.0 | 70.0 |
| 20 | 80.4 (554.3) | 29.0 | 71.0 |
| 29.8 | 106.6 (735.0) | 28.0 | 72.0 |
| 30 | 107 (737.7) | 28.0 | 72.0 |
| 40 | 140 (965.3) | 27.0 | 73.0 |
| 50 | 181 (1248) | 26.0 | 74.0 |
| 60 | 231 (1593) | 25.1 | 74.9 |
| 70 | 292 (2013) | 24.4 | 75.6 |
| 79.7 | 363 (2503) | 23.8 | 76.2 |
| 80 | 366 (2523) | 23.8 | 76.2 |
| 90 | 458 (3158) | 23.1 | 76.9 |
| 100 | 578 (3985) | 21.3 | 78.7 |
| 110 | 817 (5633) | 17.5 | 82.5 |

Based upon these findings, the present invention provides an azeotropic or azeotrope-like composition of from about 67.2 to about 82.5 mole percent HFC-1243zf and from about 32.8 to about 17.5 mole percent HF, said composition having a boiling point of from about 110°C at about 817 psia (5633 kPa) to about -40°C at about 8.0 psia (55.2 kPa).

### EXAMPLE 2

### Azeotropic distillation for the separation of HFC-1243zf from HF without an added entrainer

Example 2 demonstrates that HF may be separated from HFC-1243zf by azeotropic distillation with no added entrainer. The feed composition for this example is approximately what could be expected as the output from a reactor producing HFC-1234zf from HCC-250fb and HF, approximately 67 mol% HF and 33 mol% HFC-1243zf (29.7 wt% HF and 70.3 wt% HFC-1243zf).

Referring now to FIG 1, a composition comprising HF and HFC-1243zf is fed to the top stage of a first column 110 via stream 100. This first column contains 8 theoretical stages and is operated under appropriate conditions to approach the low-boiling HF/HFC-1243zf azeotrope. Because HF is being fed to this first column in excess of that needed to form the azeotrope with the HFC-1243zf, HF is recovered as a product stream out the bottoms of the column via stream 120, while a composition near to the HF/HFC-1243zf azeotrope is recovered as distillate via stream 130. Stream 130 is condensed in condenser 140, mixed with the nearly azeotropic composition recycled from a second column via stream 250 and the combined stream is sub-cooled in cooler 160 and sent to decanter 180 where the combined stream 170 separates into HF-rich and HFC-1243zf-rich phase fractions removed via streams 190 and 200, respectively. Stream 190 is recycled to the top stage of the first column as reflux. Stream 200 is fed to the top stage of a second distillation column 210, containing 19 theoretical stages and operated under conditions to approach the HF/HFC-1243zf azeotrope. Because HFC-1243zf is being fed to this second column in excess of that needed to form the low-boiling HF/HFC-1243zf azeotrope, HFC-1243zf is recovered as a product stream out the bottoms of the column via stream 220 while a composition close to the HF/HFC-1243zf azeotrope is recovered as distillate via stream 230. Stream 230 is condensed in condenser 240, mixed with the nearly azeotropic composition from the first column via stream 150 and fed to cooler 160 and then decanter 180.

The data in Table 2 were calculated using measured and calculated thermodynamic properties.

**TABLE 2**

| Component or variable | First dist. col. feed | First column distillate | First dist. col. bottom (HF product) | HF rich phase (from decanter) | HFC-1243zf rich phase (from decanter) | Second distillate | Second dist. col. Bottom (HFC-1243zf product) |
|---|---|---|---|---|---|---|---|
| Stream No. | **100** | **130** | **120** | **190** | **200** | **230** | **220** |
| HF, wt% | 29.7 | 11.9 | 100 | 47.2 | 2.2 | 4.3 | 1 ppm |
| HFC-1243zf, wt% | 70.3 | 88.1 | 1 ppm | 52.8 | 97.8 | 95.7 | 100 |
| Temp, °C | 30.0 | 48.0 | 102 | -40.0 | -40.0 | 45.5 | 49.0 |
| Pres, psia | 165 | 160 | 160 | 159 | 159 | 160 | 160 |
| Pres. kPa | 1137 | 1103 | 1103 | 1096 | 1096 | 1103 | 1103 |

### EXAMPLE 3 (not part of the invention)

### Azeotropic distillation for the separation of HFC-1243zf from HF using propane as the entrainer

Example 3 demonstrates that HF may be separated from HFC-1243zf by azeotropic distillation using propane as the entrainer. The same feed stream composition is used for this example as for Example 1.

Referring now to FIG 2, a composition consisting of HF and HFC-1243zf is fed to a first column 110 containing 8 theoretical stages via stream 100. An HF-rich and propane-lean composition is also fed to the top stage of column 110 via stream 190. Because the combined amount of HF in streams 100 and 190 is in excess of that needed to form the low-boiling HF/ HFC-1243zf azeotrope, HF is recovered as a product stream essentially free of both HFC-1243zf and propane from the bottom of column 110 via stream 120. A composition near the HF/ HFC-1243zf azeotrope is recovered as the distillate via stream 130. Stream 130 is condensed by condenser 140 forming stream 150 and mixed with both the condensed distillate stream 250 from a second distillation column and, as needed, additional propane added via stream 260. Combined streams 150, 250, and 260 are sent to cooler 160 and then to decanter 180 where the sub-cooled liquid stream 170 separates into HF-rich and propane-rich liquid phase fractions which are removed via streams 190 and 200, respectively. The HFC-1243zf present in the decanter primarily distributes into the propane-rich liquid phase fraction. Stream 190 is recycled to the first column. For further separation the HF-lean liquid phase fraction in the decanter is fed to the top stage of a second distillation column 210 via stream 200. Because the amount of HFC-1243zf in stream 200 is in excess of that needed to form the azeotropes, HFC-1243zf is recovered as a product stream essentially free of both HF and propane from the bottom of column 210 via stream 220. A ternary composition enriched in propane relative to stream 200 leaves the top of the second column as the distillate via stream 230. Stream 230 is condensed by condenser 240, forming stream 250, and combined with streams 150 and 260 as previously described.

The data in Table 3 were calculated using measured and calculated thermodynamic properties.

**TABLE 3**

| Component or variable | First dist. col. feed | First distillate | First dist. col. bottom (HF product) | HF rich phase (from decanter) | Propane rich phase (from decanter) | Second distillate | Second dist. col. Bottom (HFC-1243zf product) |
|---|---|---|---|---|---|---|---|
| Stream No. | **100** | **130** | **120** | **190** | **200** | **230** | **220** |
| HF, wt% | 29.7 | 10.84 | 100 | 50.1 | 1.0 | 1.4 | <1 ppm |
| HFC-1243zf, wt% | 70.3 | 88.83 | 1 ppm | 48.4 | 79.1 | 70.9 | 100 |
| Propane, wt% | 0 | 0.33 | <1 ppm | 1.5 | 19.9 | 27.7 | 1 ppm |
| Temp, °C | 30.0 | 33.8 | 88.9 | -25.0 | -25.0 | 21.4 | 36.2 |
| Pres, psia | 116 | 115 | 116 | 115 | 115 | 115 | 116 |

### EXAMPLE 4 (not part of the invention)

This Example shows one way in which HF may be separated from HFC-1243zf and HFC-254fb by azeotropic distillation using an added entrainer. The composition of the feed mixture is such as one might obtain from a dehydrofluorination reactor operated with partial conversion, i.e., it contains equimolar amounts of HF and HFC-1243zf and any unreacted HFC-254fb.

Referring now to FIG 4, a stream comprising HF, HFC-1243zf, and HFC-254fb is fed to the third stage from the top of a first distillation column 110 via stream 100. An entrainer-rich stream is also fed to this column via stream 190. In this example, propane is used as the entrainer.

Column 110 contains 19 theoretical stages and is operated under conditions to cause HF to distill overhead with the entrainer due to the existence of the low-boiling HF/propane azeotrope. Sufficient propane is fed to this first column via stream 190 such that HFC-1243zf and HFC-254fb may be obtained essentially free of propane and HF as the bottoms from column 110 via stream 120. The HFC-1243zf and HFC-254fb in stream 120 may then optionally be separated from each other by conventional distillation and the HFC-254fb optionally recycled back to a dehydrofluorination reactor to form HFC-1243zf. The distillate from column 110, removed via stream 130, contains essentially all of the propane and HF in column feeds 100 and 190 as well as some HFC-254fb and HFC-1243zf. This first distillate stream 130 is condensed by condenser 140 to form stream 150, which is then mixed with condensed distillate stream 250 from the second distillation column and, as needed, additional fresh propane added via stream 260. This combined stream is sub-cooled by cooler 160 and sent via stream 170 to decanter 180 where it separates into propane-rich and HF-rich liquid phase fractions which are removed via streams 190 and 200, respectively. The majority of the HFC-254fb and HFC-1243zf present in the decanter partition into the propane-rich phase fraction. The propane-rich phase fraction is fed to the top stage of the first distillation column 110 via stream 190 as reflux where it will undergo additional separation. The HF-rich fraction from the decanter is fed via stream 200 to the top stage of a second distillation column 210 containing 8 theoretical stages and operated under conditions such that a bottoms stream of HF essentially free of HFC-254fb, HFC-1243zf, and propane is produced and removed via stream 220. The distillate from column 210, removed via stream 230 and containing essentially all of the HFC-254fb, HFC-1243zf, and propane present in stream 200 plus the HF not recovered in product stream 220, has a composition approaching the HF/HFC-1243zf azeotrope, and is condensed by condenser 240 and removed via stream 250. Condensed distillate stream 250 is combined with both the condensed distillate stream 150 from the first column and, as needed, fresh entrainer, added via stream 260, then cooled and fed to the decanter for further separation.

The data in Table 4 were obtained by calculation using measured and calculated thermodynamic properties.

**TABLE 4**

| Component or variable | Feed | First col btm | First Dist | Propane-rich phase | H F-rich phase | Second col btm | Second Dist |
|---|---|---|---|---|---|---|---|
| Stream # | 100 | 120 | 130 | 190 | 200 | 220 | 230 |
| HF, wt% | 5.2 | <1 ppm | 3.6 | 0.5 | 60.8 | 100 | 51.9 |
| HFC-254fb, wt% | 70.1 | 73.9 | 0.4 | 0.4 | 0.2 | <1 ppm | 0.3 |
| HFC-1243zf, wt% | 24.7 | 26.1 | 62.8 | 64.7 | 37.4 | <1 ppm | 45.9 |
| propane, wt% | 0 | <1 ppm | 33.2 | 34.3 | 1.6 | <1 ppm | 1.9 |
| Temp, °C | 30.0 | 69.6 | 18.9 | -30.0 | -30.0 | 88.9 | 72.8 |
| Pres, psia | 116 | 116 | 115 | 115 | 115 | 116 | 115 |

### EXAMPLE 5 (not part of the invention)

This Example shows how HFC-254eb can be separated from a mixture comprising HF, HFC-254eb and HFC-1243zf by azeotropic distillation using an HFC-1243zf as an entrainer, followed by separation of HFC-1243zf and HF by azeotropic distillation using propane as the entrainer. The feed composition for this example is that composition expected as output from a dehydrofluorination reactor running at about 50% conversion, which comprises HF and HFC-1243zf (in equimolar amounts), and any unreacted HFC-254eb at about 33 mole percent.

Referring now to FIG 5, a stream comprising HF, HFC-1243zf, and HFC-254eb is fed to the 30^{th} stage from the top of a first distillation column 30 (containing 40 theoretical stages) via stream 10. Column 30 is operated under conditions to approach the low-boiling HF/HFC-1243zf azeotrope, which is removed from the top of the column via stream 50, condensed in condenser 60, and the condensed stream 70 is split into reflux 80 for column 30 and distillate 100, which is fed to column 110 via stream 100. This first column 30 can be designed and operated in such a way that the near azeotropic distillate is essentially free of HFC-254eb. The amount of HFC-1243zf in feed stream 10 is not sufficient to form a near-azeotropic mixture with all of the HF in stream 10. However, by recycling enough supplemental HFC-1243zf from the second column (110) bottoms to the first column via stream 20, essentially all of the HF can be distilled overhead as the HF/HFC-1243zf azeotrope such that HFC-254eb is obtained essentially free of HFC-1243zf and HF as the bottoms product from column 30 via stream 40. The HFC-254eb may then optionally be recycled back to a reactor for production of HFC-1243zf, or may be further purified and then recycled.

A propane-rich stream 190 is also fed to the top stage of this second column 110 from decanter 180. Distillation column 110 is operated under conditions such that the distillate, removed via stream 130, contains essentially all of the propane and HF in the column feeds 100 and 190 and produces an HFC-1243zf bottoms product essentially free of HF and propane which is removed via stream 120. Part of the HFC-1243zf bottoms stream 120 is recycled to the 12^{th} stage of the first column 30 via stream 20, as previously described, and the rest becomes the purified HFC-1243zf product removed via stream 125. Distillate stream 130 is condensed by condenser 140 to form stream 150, which is then mixed with the condensed distillate stream 250 from the second distillation column and, as needed, fresh propane entrainer added via stream 260. This combined stream is cooled by cooler 160 and sent via stream 170 to decanter 180 where it separates into propane-rich and HF-rich liquid phase fractions, which are removed via streams 190 and 200, respectively. The majority of the HFC-1243zf present in the decanter partitions into the propane-rich phase fraction. The decanter propane-rich fraction is fed to the top stage of column 110 via stream 190. The decanter HF-rich fraction is fed, via stream 200, to the top stage of a third distillation column 210, containing 8 theoretical stages, and operated under conditions, which produce a bottoms product consisting of HF essentially free of HFC-1243zf and propane, which is removed via stream 220. The distillate from column 210, which is removed via stream 230 and contains essentially all of the HFC-1243zf and propane present in the column feed (stream 200) and any HF not recovered in product stream 220, is condensed by condenser 240, forming stream 250. Condensed distillate stream 250 is combined with both the condensed distillate stream 150 from the second column and, as needed, fresh propane entrainer, added via stream 260, then cooled and fed to the decanter via stream 170 for further separation.

The data in Table 5 were obtained by calculation using measured and calculated thermodynamic properties.

**TABLE 5**

| Component or variable | Feed | First col btms | First col dist | Second col btms | Second dist | Propane-decanter rich phase | HF-rich decanter phase | Third col btms | Third col dist |
|---|---|---|---|---|---|---|---|---|---|
| Stream # | 10 | 40 | 100 | 120 | 130 | 190 | 200 | 220 | 230 |
| HF, wt% | 8.5 | <1 ppm | 7.7 | <1ppm | 6.0 | 0.5 | 64.1 | 100 | 11.3 |
| HFC-254eb, wt% | 50.7 | 100 | <1ppm | <1ppm | <1ppm | <1ppm | <1ppm | <1ppm | <1ppm |
| HFC-1243zf, wt% | 40.8 | 30 ppm | 92.3 | 100 | 70.4 | 74.5 | 34.8 | 1 ppm | 86.0 |
| Propane, wt% | 0 | 0 | <1ppm | 1 ppm | 23.7 | 25.0 | 1.1 | <1ppm | 2.7 |
| Temp, °C | 20.0 | 60.1 | 21.8 | 24.8 | 10.7 | -40.0 | -40.0 | 76.7 | 22.4 |
| Pressure, psia | 94.7 | 84.8 | 84.7 | 84.8 | 84.7 | 84.7 | 84.8 | 84.8 | 84.7 |

Note that not all of the activities described above in the general description or the examples are required, that a portion of a specific activity may not be required, and that one or more further activities may be performed in addition to those described. Still further, the order in which activities are listed are not necessarily the order in which they are performed.

## Claims

1. A process for separating a mixture comprising HF and HFC-1243zf, said process comprising:
a. feeding the composition comprising HF and HFC-1243zf to a first distillation column;
b. removing an azeotropic or azeotrope-like composition consisting essentially of from 67.2 to 82.5 mole percent HFC-1243zf and from 32.8 to 17.5 mole percent HF, said composition having a vapor pressure from 8.0 psia (55.2 kPa) to 817 psia (5633 kPa) at a temperature from -40°C to 110°C, as a first distillate and either i) HF or ii) HFC-1243zf, as a first column bottoms composition;
c. condensing the first distillate to form two liquid phases, being i) an HF-rich phase and ii) a HFC-1243zf-rich phase; and
d. recycling a first liquid phase enriched in the same compound that is removed as the first column bottoms, said first liquid phase being either i) HF-rich phase or ii) HFC-1243zf-rich phase, back to the first distillation column.

2. The process of claim 1, further comprising feeding a second liquid phase not recycled in step (d), said second liquid phase being either i) HF-rich phase or ii) HFC-1243zf-rich phase, to a second distillation column, and recovering the compound not recovered in step (b) as the first column bottoms composition as the second column bottoms composition.

3. A process according to claim 1 for separating HFC-1243zf from a mixture comprising hydrogen fluoride and said HFC-1243zf, wherein said HFC-1243zf is present in a concentration greater than the azeotrope concentration for hydrogen fluoride and said HFC-1243zf, said process comprising:
a. feeding said mixture comprising hydrogen fluoride and said HFC-1243zf to a first distillation column;
b. removing an azeotrope or azeotrope-like composition as defined in claim 1 as a first distillate from the first distillation column;
c. recovering HFC-1243zf containing less than 100 ppm of hydrogen fluoride from the bottom of the first distillation column;
d. condensing the azeotrope composition to form two liquid phases, being i) a hydrogen fluoride-rich phase and ii) a HFC-1243zf-rich phase; and
e. recycling the HFC-1243zf-rich phase to the first distillation column.

4. The process of claim 3 further comprising:
a. feeding the hydrogen fluoride-rich phase to a second distillation column, and
b. recovering hydrogen fluoride containing less than 100 ppm of HFC-1243zf from the bottom of the second distillation column.

5. A process according to claim 1 for separating hydrogen fluoride from a mixture comprising hydrogen fluoride and HFC-1243zf, wherein hydrogen fluoride is present in a concentration greater than the azeotrope concentration for hydrogen fluoride and said HFC-1243zf, said process comprising:
a. feeding said mixture comprising hydrogen fluoride and HFC-1243zf to a first distillation column;
b. removing an azeotrope or azeotrope-like composition as defined in claim 1 as a distillate from the first distillation column;
c. recovering hydrogen fluoride containing less than 100 ppm of HFC-1243zf from the bottom of the first distillation column;
d. condensing the azeotrope composition to form two liquid phases, being a HFC-1243zf-rich phase and a hydrogen fluoride-rich phase; and
e. recycling the HF-rich phase to the first distillation column.

6. A process for the separation of HFC-1243zf from a mixture of HFC-1243zf, HF, and at least one of HFC-254fb or HFC-254eb, said process comprising:
a) subjecting said mixture to a first distillation step, wherein additional HFC-1243zf is fed from a second distillation step, to form a first distillate comprising an azeotrope of HFC-1243zf and HF as defined in claim 1 and a first bottoms composition comprising at least one of HFC-254fb or HFC-254eb;
b) feeding said first distillate to a second distillation step to form a second distillate comprising an azeotrope of HFC-1243zf and HF as defined in claim 1 and a second bottoms composition comprising HFC-1243zf containing less than 100 ppm of HF;
c) condensing said second distillate to form two liquid phases, being i) an HF-rich phase and ii) an HFC-1243zf-rich phase; and
d) recycling the HFC-1243zf-rich phase from (c) back to the first distillation step.

7. The process of claim 6, further comprising feeding the HF-rich phase to a third distillation step to form a third distillate comprising an azeotrope of HFC-1243zf and HF and a third bottoms composition comprising HF containing less than 100 ppm of HFC-1243zf.

## Patentansprüche

1. Verfahren zum Trennen eines Gemisches, umfassend HF und HFC-1243zf, wobei das Verfahren umfasst:
a. Einspeisen der Zusammensetzung, umfassend HF und HFC-1243zf, in eine erste Destillationskolonne;
b. Entfernen einer azeotropen oder Azeotrop-ähnlichen Zusammensetzung, bestehend im Wesentlichen aus von 67,2 bis 82,5 Molprozent HFC-1243zf und von 32,8 bis 17,5 Molprozent HF, wobei die Zusammensetzung einen Dampfdruck von 8,0 psia (55,2 kPa) bis 817 psia (5633 kPa) bei einer Temperatur von -40°C bis 110°C hat, als ein erstes Destillat und entweder i) HF oder ii) HFC-1243zf als eine erste Kolonnensumpfzusammensetzung;
c. Kondensieren des ersten Destillats, um zwei flüssige Phasen zu bilden, die i) eine HF-reiche Phase und ii) eine HFC-1243zf-reiche Phase sind; und
d. Zurückführen einer ersten flüssigen Phase, angereichert an der gleichen Verbindung, die als der erste Kolonnensumpf entfernt wird, wobei die erste flüssige Phase entweder i) eine HF-reiche Phase oder ii) eine HFC-1243zf-reiche Phase ist, zurück in die erste Destillationskolonne.

2. Verfahren nach Anspruch 1, weiterhin umfassend Einspeisen einer zweiten flüssigen Phase, nicht zurückgeführt in Schritt (d), wobei die zweite flüssige Phase entweder i) eine HF-reiche Phase oder ii) eine HFC-1243zf-reiche Phase ist, in eine zweite Destillationskolonne und Zurückgewinnen der Verbindung, nicht zurückgewonnen in Schritt (b) als die erste Kolonnensumpfzusammensetzung, als die zweite Kolonnensumpfzusammensetzung.

3. Verfahren gemäß Anspruch 1 zum Abtrennen von HFC-1243zf aus einem Gemisch, umfassend Fluorwasserstoff und das HFC-1243zf, wobei das HFC-1243zf in einer Konzentration, größer als die azeotrope Konzentration für Fluorwasserstoff und das HFC-1243zf, vorhanden ist, wobei das Verfahren umfasst:
a. Einspeisen des Gemisches, umfassend Fluorwasserstoff und das HFC-1243zf, in eine erste Destillationskolonne;
b. Entfernen einer azeotropen oder Azeotrop-ähnlichen Zusammensetzung, wie in Anspruch 1 definiert, als ein erstes Destillat aus der ersten Destillationskolonne;
c. Zurückgewinnen von HFC-1243zf, enthaltend weniger als 100 ppm Fluorwasserstoff, aus dem Sumpf der ersten Destillationskolonne;
d. Kondensieren der azeotropen Zusammensetzung, um zwei flüssige Phasen zu bilden, die i) eine Fluorwasserstoff-reiche Phase und ii) eine HFC-1243zf-reiche Phase sind; und
e. Zurückführen der HFC-1243zf-reichen Phase in die erste Destillationskolonne.

4. Verfahren nach Anspruch 3, weiterhin umfassend:
a. Einspeisen der Fluorwasserstoff-reichen Phase in eine zweite Destillationskolonne, und
b. Zurückgewinnen von Fluorwasserstoff, enthaltend weniger als 100 ppm HFC-1243zf, aus dem Sumpf der zweiten Destillationskolonne.

5. Verfahren gemäß Anspruch 1 zum Abtrennen von Fluorwasserstoff aus einem Gemisch, umfassend Fluorwasserstoff und HFC-1243zf, wobei der Fluorwasserstoff in einer Konzentration, größer als die azeotrope Konzentration für Fluorwasserstoff und das HFC-1243zf, vorhanden ist, wobei das Verfahren umfasst:
a. Einspeisen des Gemisches, umfassend Fluorwasserstoff und HFC-1243zf, in eine erste Destillationskolonne;
b. Entfernen einer azeotropen oder Azeotrop-ähnlichen Zusammensetzung, wie in Anspruch 1 definiert, als einem Destillat aus der ersten Destillationskolonne;
c. Zurückgewinnen von Fluorwasserstoff, enthaltend weniger als 100 ppm HFC-1243zf, aus dem Sumpf der ersten Destillationskolonne;
d. Kondensieren der azeotropen Zusammensetzung, um zwei flüssige Phasen zu bilden, die eine HFC-1243zf-reiche Phase und eine Fluorwasserstoff-reiche Phase sind; und
e. Zurückführen der HF-reichen Phase in die erste Destillationskolonne.

6. Verfahren für die Abtrennung von HFC-1243zf aus einem Gemisch von HFC-1243zf, HF und mindestens einem von HFC-254fb oder HFC-254eb, wobei das Verfahren umfasst:
a) Unterwerfen des Gemisches einem ersten Destillationsschritt, wobei zusätzliches HFC-1243zf aus einem zweiten Destillationsschritt eingespeist wird, um ein erstes Destillat, umfassend ein Azeotrop von HFC-1243zf und HF, wie in Anspruch 1 definiert, und eine erste Sumpfzusammensetzung, umfassend mindestens eines von HFC-254fb oder HFC-254eb, zu bilden;
b) Einspeisen des ersten Destillats in einen zweiten Destillationsschritt, um ein zweites Destillat, umfassend ein Azeotrop von HFC-1243zf und HF, wie in Anspruch 1 definiert, und eine zweite Sumpfzusammensetzung, umfassend HFC-1243zf, enthaltend weniger als 100 ppm HF, zu bilden;
c) Kondensieren des zweiten Destillats, um zwei flüssige Phasen zu bilden, die i) eine HF-reiche Phase und ii) eine HFC-1243zf-reiche Phase sind; und
d) Zurückführen der HFC-1243zf-reichen Phase von (c) zurück in den ersten Destillationsschritt.

7. Verfahren nach Anspruch 6, weiterhin umfassend Einspeisen der HF-reichen Phase in einen dritten Destillationsschritt, um ein drittes Destillat, umfassend ein Azeotrop von HFC-1243zf und HF, und eine dritte Sumpfzusammensetzung, umfassend HF, enthaltend weniger als 100 ppm HFC-1243zf, zu bilden.

## Revendications

1. Procédé de séparation d'un mélange comprenant du HF et du HFC-1243zf, ledit procédé comprenant:
a. l'alimentation de la composition comprenant du HF et du HFC-1243zf au niveau d'une première colonne de distillation;
b. l'élimination d'une composition azéotrope ou type azéotrope constituée essentiellement de 67,2 à 82,5 pour cent en mole de HFC-1243zf et de 32,8 à 17,5 pour cent en mole de HF, ladite composition présentant une pression de vapeur de 8,0 lb/po² absolue (55,2 kPa) à 817 lb/po² absolue (5 633 kPa) à une température de -40°C à 110°C, comme premier distillat et soit i) du HF soit ii) du HFC-1243zf, comme première composition de fond de colonne;
c. la condensation du premier distillat pour former deux phases liquides, étant i) une phase riche en HF et ii) une phase riche en HFC-1243zf; et
d. le recyclage d'une première phase liquide enrichie du même composé que celui retiré en tant que premier fond de colonne, ladite première phase liquide étant soit i) une phase riche en HF soit ii) une phase riche en HFC-1243zf, de retour vers la première colonne de distillation.

2. Procédé selon la revendication 1, comprenant en outre l'alimentation d'une seconde phase liquide non recyclée dans l'étape (d), ladite seconde phase liquide étant soit i) la phase riche en HF soit ii) la phase riche en HFC-1243zf, au niveau d'une seconde colonne de distillation, et la récupération du composé non récupéré dans l'étape (b) comme la première composition de fond de colonne comme seconde composition de fond de colonne.

3. Procédé selon la revendication 1 de séparation de HFC-1243zf d'un mélange comprenant du fluorure d'hydrogène et ledit HFC-1243zf, où ledit HFC-1243zf est présent en une concentration supérieure à la concentration azéotrope pour le fluorure d'hydrogène et ledit HFC-1243zf, ledit procédé comprenant:
a. l'alimentation dudit mélange comprenant du fluorure d'hydrogène et ledit HFC-1243zf au niveau d'une première colonne de distillation;
b. l'élimination d'une composition azéotrope ou type azéotrope telle que définie selon la revendication 1 sous la forme d'un premier distillat à partir de la première colonne de distillation;
c. la récupération de HFC-1243zf contenant moins de 100 ppm de fluorure d'hydrogène du fond de la première colonne de distillation;
d. la condensation de la composition azéotrope pour former deux phases liquides, étant i) une phase riche en fluorure d'hydrogène et ii) une phase riche en HFC-1243zf; et
e. le recyclage de la phase riche en HFC-1243zf au niveau de la première colonne de distillation.

4. Procédé selon la revendication 3 comprenant en outre:
a. l'alimentation de la phase riche en fluorure d'hydrogène au niveau d'une seconde colonne de distillation, et
b. la récupération du fluorure d'hydrogène contenant moins de 100 ppm de HFC-1243zf du fond de la seconde colonne de distillation.

5. Procédé selon la revendication 1 de séparation de fluorure d'hydrogène d'un mélange comprenant du fluorure d'hydrogène et du HFC-1243zf, où le fluorure d'hydrogène est présent en une concentration supérieure à la concentration azéotrope pour le fluorure d'hydrogène et ledit HFC-1243zf, ledit procédé comprenant:
a. l'alimentation dudit mélange comprenant du fluorure d'hydrogène et du HFC-1243zf au niveau d'une première colonne de distillation;
b. l'élimination d'une composition azéotrope ou type azéotrope telle que définie selon la revendication 1 comme distillat à partir de la première colonne de distillation;
c. la récupération du fluorure d'hydrogène contenant moins de 100 ppm de HFC-1243zf du fond de la première colonne de distillation;
d. la condensation de la composition azéotrope pour former deux phases liquides, étant une phase riche en HFC-1243zf et une phase riche en fluorure d'hydrogène; et
e. le recyclage de la phase riche en HF au niveau de la première colonne de distillation.

6. Procédé de séparation de HFC-1243zf d'un mélange de HFC-1243zf, HF, et d'au moins l'un parmi le HFC-254fb ou le HFC-254eb, ledit procédé comprenant:
a) la soumission dudit mélange à une première étape de distillation, où du HFC-1243zf supplémentaire est alimenté depuis une seconde étape de distillation, pour former un premier distillat comprenant un azéotrope de HFC-1243zf et de HF tels que définis selon la revendication 1 et une première composition de fond comprenant au moins l'un du HFC-254fb ou du HFC-254eb;
b) l'alimentation dudit premier distillat au niveau d'une seconde étape de distillation pour former un second distillat comprenant un azéotrope de HFC-1243zf et HF tels que définis selon la revendication 1 et une seconde composition de fond comprenant du HFC-1243zf contenant moins de 100 ppm de HF;
c) la condensation dudit second distillat pour former deux phases liquides, étant i) une phase riche en HF et ii) une phase riche en HFC-1243zf; et
d) le recyclage de la phase riche en HFC-1243zf de (c) de retour vers la première étape de distillation.

7. Procédé selon la revendication 6, comprenant en outre l'alimentation de la phase riche en HF au niveau d'une troisième étape de distillation pour former un troisième distillat comprenant un azéotrope de HFC-1243zf et de HF et une troisième composition de fond comprenant du HF contenant moins de 100 ppm de HFC-1243zf.
